# EUROPEAN PATENT APPLICATION

(11) **EP 3 235 538 A1**
(43) Date of publication of application: **25.10.2017**
(21) Application number: 15869985.0
(22) Date of filing: 15.12.2015
(51) Int. Cl.: A61M 37/00, B81B 1/00

(54) **MICRONEEDLE PATCH, METHOD FOR MANUFACTURING SAME, AND APPARATUS FOR MANUFACTURING MICRONEEDLE ARRAY**

(30) Priority: 15.12.2014 JP 2014252907
(71) Applicant: Labo Juversa Co., Ltd., Sapporo-shi, Hokkaido 060-0001 (JP); BioSerenTach Co., Ltd., Kyoto-shi, Kyoto 604-0874 (JP)
(72) Inventor: ONO, Ichiro, Sapporo-shi, Hokkaido 060-0001 (JP); YAMADA, Shinya, Kyoto-shi, Kyoto 604-8551 (JP); CHIYAMA, Masateru, Kyoto-shi, Kyoto 604-8551 (JP); AKITA, Kensaku, Kyoto-shi, Kyoto 604-8551 (JP); UENO, Takako, Kyoto-shi, Kyoto 604-8551 (JP); IDE, Yuko, Kyoto-shi, Kyoto 604-8551 (JP); NAGAI, Sachi, Kyoto-shi, Kyoto 604-8551 (JP); AKAO, Osanobu, Kyoto-shi, Kyoto 604-8551 (JP); TAKADA, Kanji, Kyoto-shi, Kyoto 600-8040 (JP)
(74) Representative: Hamer, Christopher K.
(86) International application number: PCT/JP2015/085107
(87) International publication number: WO 2016/098780

(57) **Abstract**

A microneedle patch with which a treatment is reliably completed in a short time when a microneedle is administered into skin or mucous membrane is provided. A microneedle patch (100F) includes a base member (101) and a plurality of microneedles (103g) supported by the base member (101). Each microneedle (103g) includes a top-section layer (104) comprising a biologically active substance to be inserted in dermis (310), and an intermediate layer (106) provided between the top-section layer (104) and the base member (101), having a composition having breaking strength weaker than breaking strength of a composition of the top-section layer (104), and having a thickness of 5 µm and 100 µm inclusive.

## Description

### Technical Field

The present invention relates to a microneedle patch capable of achieving fast and reliable drug administration by improving performance of a microneedle array for causing predetermined drug to intradermally reach into dermis and improving a breaking property of the drug at administration, and a method for manufacturing the same.

### Background Art

Recently, microneedles have been increasingly used in, for example, fields related to the medical field and beauty, cosmetic and health care. For example, drug is administered through the body surface of a human body, such as skin and mucous membrane, by using a microneedle array consisting of a plurality of microneedles. Examples of methods for manufacturing such a microneedle array include a known method of filling, with needle raw material, a plurality of recesses included in a mold by using a squeegee, and solidifying the needle raw material by drying, as disclosed in patent document 1 (Japanese unexamined Patent Application Publication No. 2012-200572).

### Prior Art Document

### Patent Document

[Patent Document 1] Japanese unexamined Patent Application Publication No. 2012-200572

### Summary of the Invention

### Object to be Solved by the Invention

The present invention provides a technology of fine and mass manufacturing of a microneedle array having highly accurate appearance and accuracy by an inkjet method, and details of an excellent mass production technology of controlling the internal structure of a microneedle so that, when administered to skin or mucous membrane, the microneedle breaks at an end of a drug containing part, which is closer to a bottom-section layer, immediately after inserted into skin, and leaves a top section as the drug containing part in target epidermis or dermis to reliably achieve an initial purpose so that a treatment is reliably completed in a short time.

### Means to Solve the Object

The present investors have intensively studied to solve the problem and have produced a microneedle capable of administering contained drug to a target site appropriately by controlling the shape of a boundary of a bottom-section layer and manufacturing an intermediate layer having strength clearly different from those of other sites and locally having a high breaking property. When the intermediate layer includes two layers, a first intermediate layer is formed of a raw material having a high hardness and a low absorbability, and a second intermediate layer is formed of a raw material having relatively low hardness and high absorbability, which intends to cause a top-section layer containing released drug to be reliably left inside dermis. A microneedle array manufacturing apparatus including a plurality of the droplet discharging apparatuses each capable of discharging a raw material obtained by changing, as appropriate, a mixture ratio and concentration of a bioabsorbable formulation that forms a microneedle to achieve the present invention was developed, and an apparatus exploiting a high technology and combined with a control device configured to control the operation state thereof was achieved. In this case, the raw material for the intermediate part may or may not include drug.

The following describes a plurality of embodiments as means for solving the above-described problems. These embodiments may be optionally combined as necessary.

To achieve the present invention, a microneedle array manufacturing apparatus according to an aspect of the present invention includes a plurality of the droplet discharging apparatuses capable of discharging raw material obtained by changing, as appropriate, a mixture ratio and concentration of a bioabsorbable formulation that forms a microneedle.

A microneedle patch according to an aspect of the present invention is a microneedle patch produced by a droplet discharging apparatus capable of separately discharging, as raw material liquid, for example, first liquid, second liquid, third liquid, and fourth liquid containing components different from each other, wherein an intermediate layer having a breaking property higher than a breaking property of a top-section layer comprising a biologically active material, having a function to prevent backflow of, through a penetrating hole, drug released in dermis, and having a thickness of 5 µm to 100 µm is formed after discharge, drying, and hardening of the top-section layer, and the intermediate layer is configured to break in the dermis in 5 seconds to 20 seconds approximately.

A microneedle patch according to another aspect of the present invention is a microneedle patch produced by discharging raw material liquid from a droplet discharging apparatus, wherein the strength of a microneedle is highest in a bottom-section layer, followed in order by a top section comprising drug, and an intermediate layer set as a layer having a lowest strength.

A microneedle patch according to yet another aspect of the present invention is a microneedle patch produced by discharging raw material liquid from a droplet discharging apparatus, wherein a weight-average molecular weight of a microneedle is largest in a top section comprising drug, followed in order by a bottom-section layer, and an intermediate layer set as a layer having a lowest weight-average molecular weight.

The microneedle patch may be a microneedle patch produced by discharging different kinds of raw material liquid from a droplet discharging apparatus, wherein the intermediate layer includes one or more layers.

A microneedle patch according to yet another aspect of the present invention is a microneedle patch produced by discharging different kinds of raw material liquid from a droplet discharging apparatus and including two or more intermediate layers, wherein a first intermediate layer is formed of a polymer material having a high hardness and a low absorbability and selected as a raw material selected to have high concentration, a dissolution time of the first intermediate layer is controlled to be 10 minutes to 24 hours, and drug contained in a top section and released in dermis is prevented from flowing back in a direction toward epidermis through a penetrating hole.

The microneedle patch produced by discharging different kinds of raw material liquid from a droplet discharging apparatus may include two or more intermediate layers, wherein a second intermediate layer is formed of a polymer material having a low hardness and a high absorbability and selected as a raw material selected to have low concentration, and a function to easily break and separate a top section from a bottom-section layer and reliably leave the top section in dermis in a short time is provided.

A microneedle patch according to yet another aspect of the present invention is a microneedle patch produced by discharging raw material liquid from a droplet discharging apparatus, wherein the microneedle patch is produced integrally with a bonding surface and a flat surface adhering to a substrate when part or all of a bottom-section layer is produced, collapse of microneedles due to piercing is prevented, a top section containing drug easily and reliably breaks from the bottom-section layer, and a plurality of intermediate layers each having a function to prevent backflow of the drug from inside of dermis are provided.

A microneedle patch according to yet another aspect of the present invention is a microneedle patch produced by discharging raw material liquid from a droplet discharging apparatus, wherein an intermediate layer is provided between a top section and a bottom-section layer, and the intermediate layer is configured to break in dermis in 5 seconds to 20 seconds approximately to leave the top section in the dermis.

The microneedle patch may be a microneedle patch produced by discharging raw material liquid from a droplet discharging apparatus, wherein an intermediate layer is inside a bottom-section layer.

A microneedle array manufacturing apparatus according to an aspect of the present invention is a microneedle array manufacturing apparatus for shaping a microneedle array consisting of a plurality of microneedles by filling a plurality of recesses formed in a mold with raw material liquid for forming the microneedles, the apparatus comprising: at least one droplet discharging apparatus capable of discharging, to each recess, a droplet of the raw material liquid in a predetermined amount equal to or smaller than the volume of the recess; and a positioning apparatus capable of adjusting relative positions of the droplet discharging apparatus and the mold so as to land the droplet into the recess from the droplet discharging apparatus, wherein the at least one droplet discharging apparatus are a plurality of droplet discharging apparatuses each capable of discharging raw material obtained by changing, as appropriate, a mixture ratio and concentration of a bioabsorbable formulation that forms a microneedle.

A microneedle array manufacturing method according to an aspect of the present invention is a microneedle patch manufacturing method comprising the processes of: forming a top-section layer in a recess of a mold; discharging, onto the top-section layer in the recess, a plurality of droplets of first intermediate-layer raw material liquid from a droplet discharging apparatus; hardening the second intermediate-layer raw material liquid to form an intermediate layer having a breaking strength weaker than a breaking strength of the top-section layer; discharging, onto the intermediate layer in the recess, a plurality of droplets of bottom-section-layer raw material liquid from the droplet discharging apparatus; and hardening the bottom-section-layer raw material liquid to form a bottom-section layer having a breaking strength stronger than the breaking strength of the intermediate layer.

### Effect of the Invention

A microneedle patch according to the present invention can achieve fine and mass manufacturing of a microneedle array having highly accurate appearance and accuracy by an inkjet method, can reliably achieve an initial purpose so that a treatment is reliably completed in a short time when microneedles are administered to skin or mucous membrane, because each microneedle breaks at a stump of a top section as a drug containing part, which is closer to a bottom-section layer, immediately after insertion into the skin to leave the top section as a drug containing part in target epidermis or dermis, and can achieve excellent mass productivity.

### Brief Description of Drawings

[Figure 1] Schematic perspective view illustrating the outline of an apparatus for manufacturing a microneedle array according to a first embodiment.
[Figure 2] Block diagram for description of a control system of the apparatus for manufacturing a microneedle array in Figure 1.
[Figure 3] Perspective view illustrating an exemplary product including a microneedle array according to the first embodiment.
[Figure 4] Partially enlarged perspective view of part of Figure 3.
[Figure 5] Perspective view illustrating an exemplary mold according to the first embodiment.
[Figure 6] Flowchart of an exemplary method for manufacturing the microneedle array according to the first embodiment.
[Figure 7] Schematic cross-sectional view for description of discharge of a droplet toward recesses.
[Figure 8] Schematic enlarged cross-sectional view for description of landing of droplets on a recess.
[Figure 9] (a) Schematic cross-sectional view illustrating a state before assembly through an assembly process, (b) Schematic cross-sectional view illustrating a state during the assembly through the assembly process, and (c) Schematic cross-sectional view illustrating a state after completion of the assembly through the assembly process.
[Figure 10] Schematic cross-sectional view for description of discharge of droplets toward recesses in a second embodiment.
[Figure 11] Perspective view illustrating an exemplary product including a microneedle array according to the second embodiment.
[Figure 12] Partially enlarged perspective view of part of Figure 11.
[Figure 13] Perspective view illustrating another exemplary product including the microneedle array according to the second embodiment.
[Figure 14] Partially enlarged perspective view of part of Figure 13.
[Figure 15] (a) Schematic cross-sectional view for description of a microneedle array according to a modification 2C, (b) Conceptual diagram for description of an exemplary product including the microneedle array according to the modification 2C, and (c) Conceptual diagram for description of another exemplary product including the microneedle array according to the modification 2C.
[Figure 16] Conceptual diagram for description of an apparatus for manufacturing a microneedle array according to a third embodiment.
[Figure 17] (a) Schematic cross-sectional view for description of a method for manufacturing a microneedle array according to a modification 1C, and (b) Conceptual diagram for description of an exemplary product including the microneedle array according to the modification 1C.
[Figure 18] (a) Schematic cross-sectional view for description of a method for manufacturing a conventional microneedle array, (b) Schematic cross-sectional view illustrating a process of manufacturing the conventional microneedle array, (c) Schematic cross-sectional view of a mold in which a conventional top-section layer is formed, (d) Schematic cross-sectional view for description of a process of filling the conventional microneedle array, and (e) Schematic cross-sectional view illustrating a process of fixing the conventional microneedle array.
[Figure 19] Schematic cross-sectional view illustrating a process of forming a top-section layer of a microneedle patch.
[Figure 20] Schematic cross-sectional view illustrating a process of forming an intermediate layer of the microneedle patch.
[Figure 21] Schematic cross-sectional view illustrating a process of forming the intermediate layer of the microneedle patch and a state after the formation.
[Figure 22] Schematic cross-sectional view illustrating a process of forming a bottom-section layer of the microneedle patch.
[Figure 23] Schematic cross-sectional view illustrating a state in which the microneedle patch is inserted.
[Figure 24] Schematic cross-sectional view illustrating a state when the microneedle patch is removed.
[Figure 25] Schematic cross-sectional view for description of dissolution of a microneedle.
[Figure 26] Schematic cross-sectional view for description of dissolution of the intermediate layer of the microneedle.
[Figure 27] Schematic cross-sectional view illustrating a process of forming a top-section layer of a microneedle patch.
[Figure 28] Schematic cross-sectional view illustrating a process forming a first intermediate layer of the microneedle patch.
[Figure 29] Schematic cross-sectional view illustrating a process of forming a second intermediate layer of the microneedle patch.
[Figure 30] Schematic cross-sectional view illustrating a process of forming a bottom-section layer of the microneedle patch.
[Figure 31] Schematic cross-sectional view illustrating a state in which the microneedle patch is inserted.
[Figure 32] Schematic cross-sectional view illustrating a state when the microneedle patch is removed.
[Figure 33] Schematic cross-sectional view for description of dissolution of a microneedle.
[Figure 34] Schematic cross-sectional view for description of dissolution of the intermediate layer of the microneedle.

### Mode of Carrying Out the Invention

The present invention is related to an apparatus for manufacturing a microneedle array, a method for manufacturing a microneedle array having an optimized breaking property to allow fast drug administration, and a product including the microneedle array.

### <First embodiment>

The following describes an apparatus for manufacturing a microneedle array, a method for manufacturing the microneedle array, and a product including the manufactured microneedle array according to a first embodiment of the present invention with reference to the accompanying drawings.

### (1) Outline of apparatus for manufacturing microneedle array

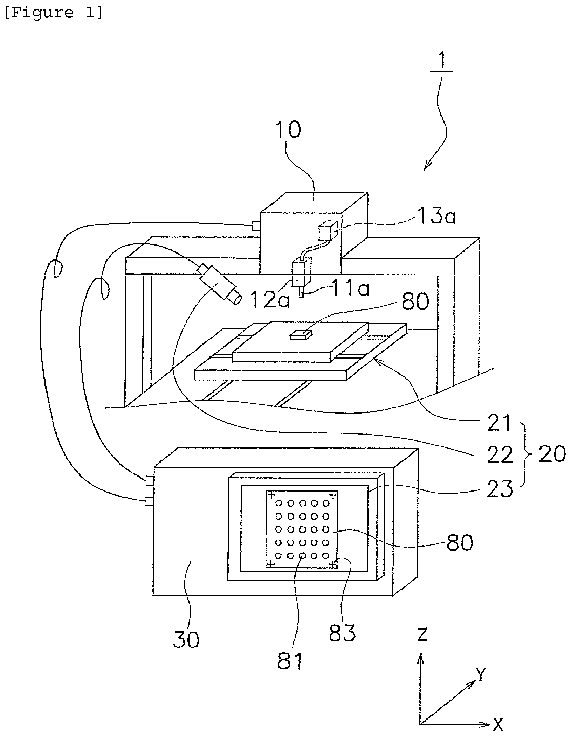

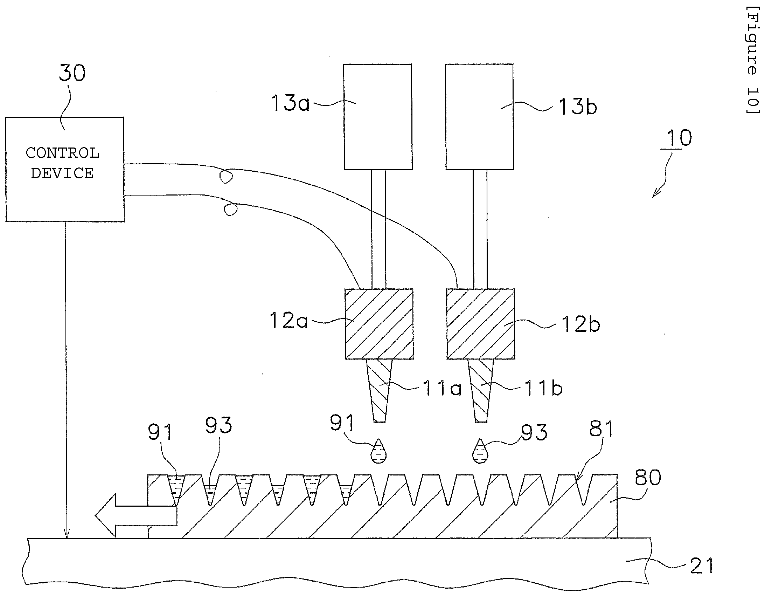

Figure 1 is a schematic perspective view illustrating the outline of the apparatus for manufacturing a microneedle array. As illustrated in Figure 1, the microneedle array manufacturing apparatus 1 includes a droplet discharging apparatus 10 and a positioning apparatus 20. The positioning apparatus 20 includes an XYZ stage 21, a CCD camera 22, and an alignment monitor 23. As illustrated in Figure 1, the droplet discharging apparatus 10 is provided with a nozzle 11a for discharging droplets, and a cartridge 13a containing raw material liquid supplied to the nozzle 11a. Although not illustrated in Figure 1, the droplet discharging apparatus 10 also includes another nozzle 11b and another cartridge 13b illustrated in Figure 10.

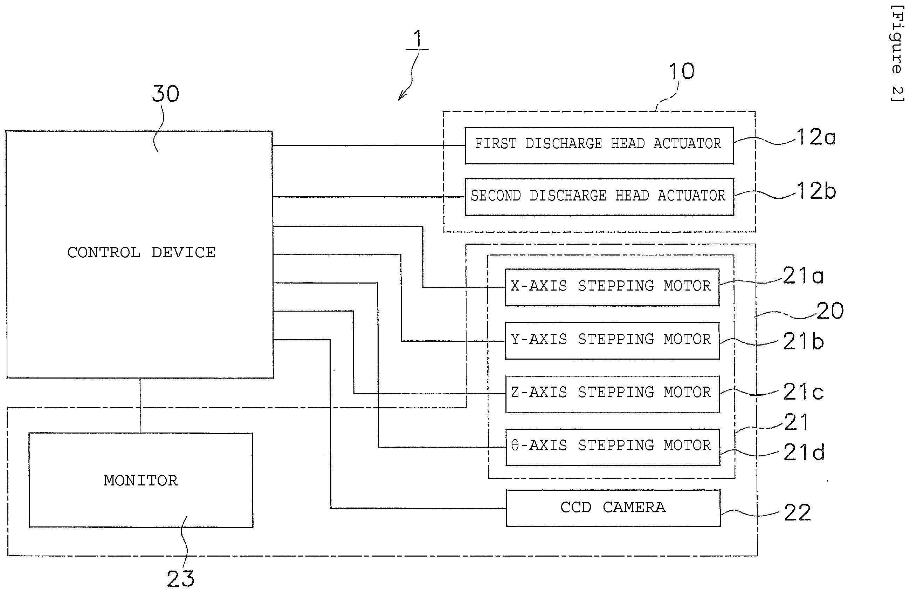

In a control system of the microneedle array manufacturing apparatus 1, as illustrated in Figure 2, the microneedle array manufacturing apparatus 1 includes a control device 30 configured to control the droplet discharging apparatus 10 and the positioning apparatus 20. In the droplet discharging apparatus 10, the control device 30 controls a first discharge head actuator 12a and a second discharge head actuator 12b. In the microneedle array manufacturing apparatus 1, the control device 30 controls the first discharge head actuator 12a and the second discharge head actuator 12b to finely adjust the number of droplets discharged from the nozzles 11a and 11b. In the positioning apparatus 20, the control device 30 controls an X-axis stepping motor 21a, a Y-axis stepping motor 21b, a Z-axis stepping motor 21c, and a θ-axis stepping motor 21d of the XYZ stage 21, the CCD camera 22, and the alignment monitor 23. A mold 80 placed on the XYZ stage 21 is moved in an X-axis direction by the X-axis stepping motor 21a, in a Y-axis direction by the Y-axis stepping motor 21b, and in a Z-axis direction by the Z-axis stepping motor 21c, and rotated about a central axis extending in the vertical direction (Z-axis direction) at the center of the XYZ stage 21 by the θ-axis stepping motor 21d.

### (2) Product including microneedle array

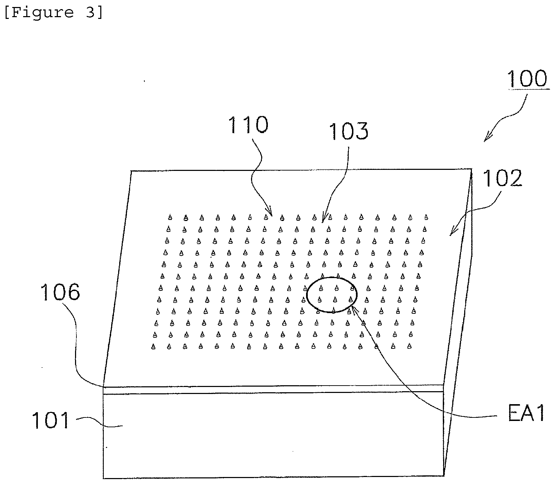

The following describes a product including a microneedle array manufactured by using the microneedle array manufacturing apparatus 1. The microneedle array manufacturing apparatus 1 forms a microneedle array 110 consisting of a plurality of microneedles 103 illustrated in Figure 3.

Each microneedle 103 is set to have, for example, a height of 10 µm to 1 mm, a maximum bottom-surface width of 10 µm to 1 mm, and an aspect ratio of 0.5 to 4.

An interval d1 between the microneedles 103 adjacent to each other (distance between nearest places on a surface 102) is set to be, for example, 10 µm to 2 mm. The microneedles 103 included in the microneedle array 110 are set to have a density of, for example, several microneedles to 10⁵ microneedles for one square centimeter approximately. To manufacture such a microneedle array 110, the microneedle array manufacturing apparatus 1 is capable of repeating across a travel distance equal to or smaller than the interval d1 of the microneedles 103. Error in the travel distance of the microneedle array manufacturing apparatus 1 is set to be smaller than the maximum bottom-surface width of each microneedle 103.

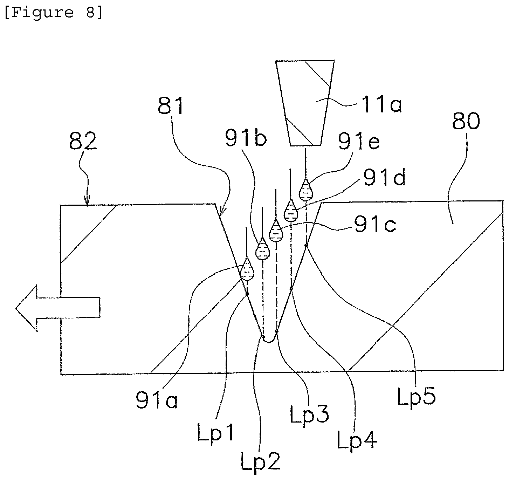

The microneedle array 110 is fixed to the surface 102 of a plate base member 101. The base member 101 has an outer dimension of, for example, 2 mm × 17 mm × 17 mm approximately. To achieve the fixation of the microneedle array 110 to the surface 102, a lamination film 109 having a composition same as that of a bottom-section layer 105 is formed on the surface 102 of the base member 101. In this manner, a product 100 including the microneedle array 110 fixed to the base member 101 is formed. When each microneedle 103 has a spired leading end part, a section of the leading end part taken along the vertical direction is angled at, for example, 30°. In this manner, when the microneedle 103 has the spired leading end part, a recess 81 (refer to Figure 8) on which droplets land has a tilted wall, which facilitates formation of the recess 81 having a shape suitable to be filled with droplets. The landing of a droplet on the recess 81 means hitting and adhesion of the droplet to the surface of the wall of the recess 81.

The plate base member 101 allows ventilation and is, for example, a porous base member. Examples of porous base members include a porous base member mainly made of cellulose acetate, a porous ceramic base member, a porous metal base member, a pulp molded product obtained by forming pulp in a plate shape, and a porous resin base member.

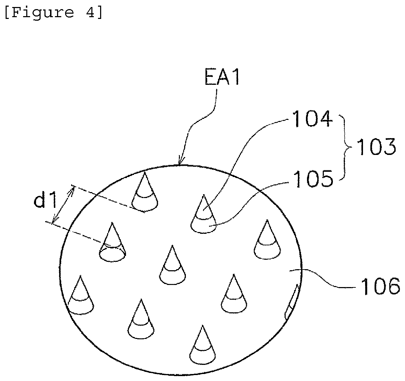

Figure 4 illustrates a partial region EA1 of Figure 3 in an enlarged manner. Each microneedle 103 has a two-layer structure consisting of a top-section layer 104 at a leading end and the bottom-section layer 105 therebelow. The top-section layer 104 and the bottom-section layer 105 have compositions different from each other.

In the following description of a component of raw material liquid, the component does not necessarily need to be dissolved in the raw material liquid. For example, when the raw material liquid is suspended liquid, the suspended liquid may have a component of, for example, microcapsules or liposomes.

### (3) Mold

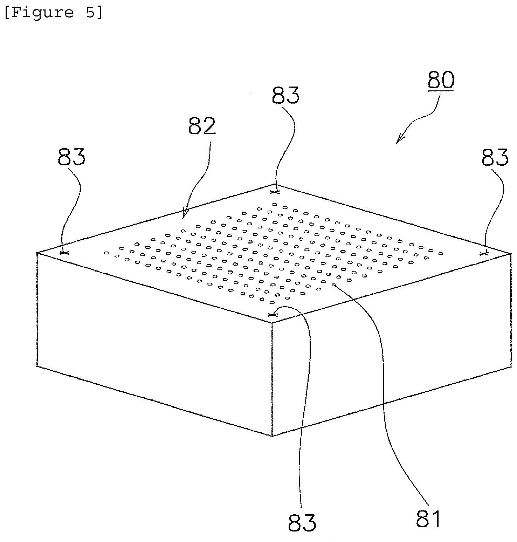

The mold 80 illustrated in Figure 5 only needs to be formed of a material hygienic against the raw material liquid, but preferably has high gas permeability. For example, the mold 80 may be formed of plastic, elastomer, ceramic, or metal. The mold 80 is preferably formed of silicone rubber. The plastic of which the mold 80 is formed is preferably, for example, polymethylpentene (TPX (registered trademark)) or polytetrafluoroethylene. The metal of which the mold 80 is formed is preferably, for example, stainless steel, which does not transmit gas but is unlikely to rust. A horizontal section of the recess 81 of the mold 80 along a surface 82 of the mold 80 has, for example, a circular shape, a polygonal shape, or an elliptical shape. The recess 81 includes an internal space having, for example, a circular cone shape, a pyramid shape, a cylindrical shape, or a rectangular column shape.

Alignment marks 83 are formed on the surface 82 of the mold 80. The alignment marks 83 are read by the CCD camera 22 of the microneedle array manufacturing apparatus 1. The alignment marks 83 are used as references to control landing of a droplet discharged from the droplet discharging apparatus 10 in the recess 81, and thus the position of each recess 81 is determined with reference to the alignment marks 83. The alignment marks 83 are hygienic and formed as, for example, bumps on the surface 82.

The mold 80 when formed of silicone rubber has an outer dimension of, for example, 6 mm × 20 mm × 20 mm, and the recesses 81 are formed in a region having a size of, for example, 15 mm × 15 mm.

### (4) Raw material liquid

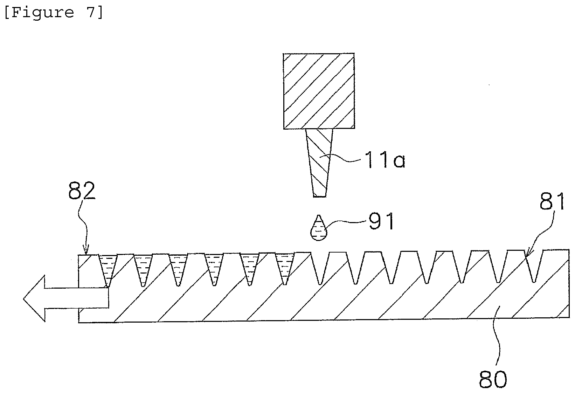

Top-section-layer raw material liquid 91 (refer to Figure 7) for forming the top-section layer 104 of each microneedle 103 is, for example, solution of a solid raw material in water, a mixed solvent of water and alcohol, or another solvent, or suspension liquid of a solid raw material in water, a mixed solvent of water and alcohol, or another solvent, or a mixture of the solution and the suspension liquid. The solid raw material is a polymer substance harmless to a human body and is, for example, a resin harmless to a human body, a polysaccharide harmless to a human body, a protein harmless to a human body, or a compound thereof harmless to a human body. Examples of compounds to be introduced into a human body include a biologically active substance used for treatment, diagnosis, and prevention of injuries and diseases.

The top-section-layer raw material liquid is, for example, a solvent of aqueous polysaccharide (comprising derivatives and salts thereof) containing a biologically active substance administered for diagnosis, treatment, and prevention of diseases. Such a solvent of the top-section-layer raw material liquid is evaporated to form, in a substrate of polysaccharide, the top-section layer 104 comprising the biologically active substance. Examples of the aqueous polysaccharide (comprising derivatives and salts thereof) include sodium chondroitin sulfate, hyaluronic acid, dextran, and carboxymethyl cellulose. Examples of the biologically active substance include insulin and growth hormone.

Bottom-section-layer raw material liquid for forming the bottom-section layer 105 of the microneedle 103 is different from the top-section-layer raw material liquid in a composition of at least one of solid raw material and solvent. When the top-section-layer raw material liquid and the bottom-section-layer raw material liquid have different compositions in this manner, the top-section layer 104 and the bottom-section layer 105 of the microneedle 103 have different compositions. The present embodiment describes exemplary medical usage of the microneedle 103 in which the top-section layer 104 comprises a biologically active medicinal substance and the bottom-section layer 105 comprises no biologically active substance. However, for example, in medical usage of the microneedle 103, the top-section layer 104 and the bottom-section layer 105 may both comprise biologically active medicinal substances. Differences can be obtained between a medical effect provided by the top-section layer 104 and the duration thereof and a medical effect provided by the bottom-section layer and the duration thereof, by providing differences between the kind and amount of the biologically active substance comprised in the top-section layer 104 and the kind and amount of the biologically active substance comprised in the bottom-section layer 105. In medical usage of the product 100 including the microneedle array, the product 100 is applicable to various kinds of drug administration when each microneedle 103 has a two-layer structure in this manner.

The top-section-layer raw material liquid is discharged as a droplet from, for example, the droplet discharging apparatus 10, and the amount of the droplet is set to be, for example, 0.1 nanoliter/droplet to 1 microliter/droplet. For example, when each recess 81 for forming one microneedle 103 has a capacity of 20 nanoliters, one droplet has 1 nanoliter to fill the recess 81 with 20 droplets. Filling with such a minute droplet preferably requires a low viscosity such as, 0.1 mPa•sec to 100 mPa•sec, preferably 1 mPa•sec to 10 mPa•sec.

### (5) Method for manufacturing product including microneedle array

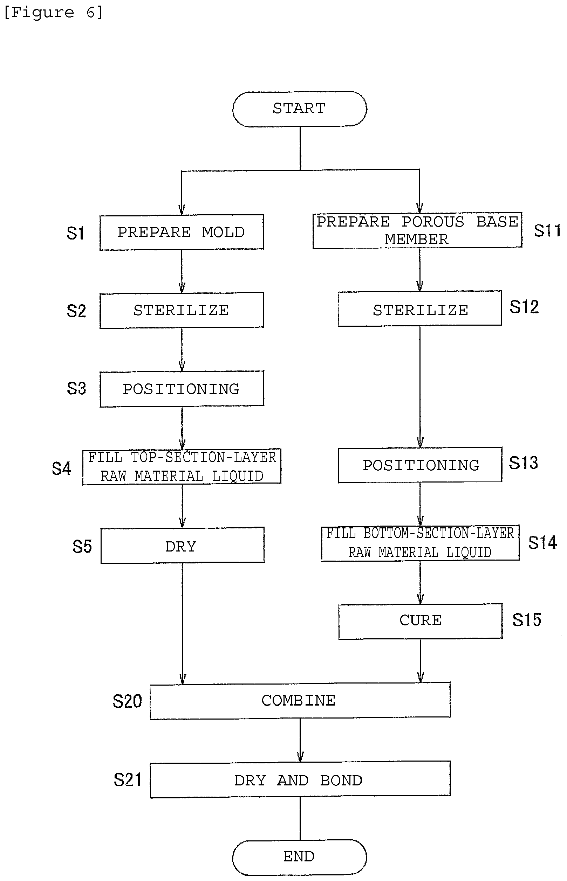

Figure 6 is a flowchart for description of a process of manufacturing the product 100 including the above-described microneedle array by using the microneedle array manufacturing apparatus 1. In the process of manufacturing the product 100 including the microneedle array, an operation from step S1 to step S5 and an operation from step S11 to step S15 in Figure 6 can be performed in parallel independently from each other. However, the two operations may share any operation if possible. Although operation of the microneedle array manufacturing apparatus 1 at each step is controlled by the control device 30, the following description omits part of description of control of each component of the microneedle array manufacturing apparatus 1 by the control device 30. In the first embodiment, only the first discharge head actuator 12a is used to discharge droplets from the nozzle 11a, whereas a manufacturing method using the second discharge head actuator 12b will be described in a second embodiment. In the first embodiment, the θ-axis stepping motor 21d is not used, whereas a manufacturing method using the θ-axis stepping motor 21d will be described in a third embodiment.

An operation using the mold 80 is performed at steps S1 to S5 in Figure 6. First, the mold 80 illustrated in Figure 5 is prepared (step S1). In the process of preparing the mold 80 at step S1, for example, a predetermined number of molds 80 are washed by water and arranged at a predetermined place. The prepared molds 80 are all sterilized through, for example, an autoclave (not illustrated) (step S2). The sterilized molds 80 are placed on the XYZ stage 21 of the microneedle array manufacturing apparatus 1 in a clean environment, and subjected to positioning (step S3).

The positioning of each sterilized mold 80 is performed after the mold 80 is placed on the XYZ stage 21 by, for example, a sterilized robotic arm. The CCD camera 22 captures an image of the alignment marks 83 of the mold 80 on the XYZ stage 21, and the control device 30 recognizes the alignment marks 83 as references, thereby performing the positioning. The control device 30 specifies the position of each recess 81 with reference to the alignment marks 83 of the mold 80, which allows the XYZ stage 21 to move the mold 80 relative to the nozzle 11a of the droplet discharging apparatus 10 so that the nozzle 11a of the droplet discharging apparatus 10 unicursally and sequentially follows the adjacent recesses 81.

At step S4, as illustrated in Figures 7 and 8, the mold 80 is moved relative to the nozzle 11a so that a droplet discharged from the nozzle 11a directly lands in each recess 81 of the mold 80 to fill the recess 81 with the top-section-layer raw material liquid 91. As illustrated in Figure 8, when small droplets land in the recess 81, a larger part of the droplets contacts air, and as a result, the top-section-layer raw material liquid 91 can be dried in a shorter time. The droplet discharging apparatus 10 and the XYZ stage 21 are controlled to operate in synchronization so that the top-section-layer raw material liquid 91 discharged from the nozzle 11a does not land on the surface 82 of the mold 80. Figure 8 illustrates five droplets 91a, 91b, 91c, 91d, and 91e of the top-section-layer raw material liquid 91 discharged from the nozzle 11a for each recess 81, and landing points Lp1, Lp2, Lp3, Lp4, and Lp5 of the respective droplets 91a, 91b, 91c, 91d, and 91e. The landing points Lp1, Lp2, Lp3, Lp4, and Lp5 are at positions different from each other in the recess 81. For example, when the droplets are sequentially discharged while the nozzle 11a is moved relative to the mold 80 at a constant speed, the landing points Lp1, Lp2, Lp3, Lp4, and Lp5 are different from each other. When the discharging is repeated to cause droplets to land on different positions while the nozzle 11a is moved in this manner, each mold 80 can be filled in a shorter time, and accordingly, a product including the microneedle array can be manufactured in a short time. The speed of the nozzle 11a relative to the mold 80 may be set to change between start and completion of the landing on each recess 81.

The number of droplets of the discharged top-section-layer raw material liquid 91 is not limited to five but may be set as appropriate. The number of droplets for each recess 81 is set to be, for example, one to several tens. The amount of droplets of the discharged top-section-layer raw material liquid 91 may be set as appropriate. For example, the amounts of the droplets 91a, 91b, 91c, 91d, and 91e may be identical to each other or different from each other. For example, the amount of droplets may be reduced at a position closer to the edge of the recess 81 and increased at a position closer to the center of the recess 81, or the amount of droplets may be increased at a position closer to the edge of the recess 81 and reduced at a position closer to the center of the recess 81, the amount of droplets may be reduced toward the end of discharging at the recess 81, or the amount of droplets may be increased toward the end of discharging at the recess 81.

In this example, the total volume of the five droplets of the top-section-layer raw material liquid 91 discharged into each recess 81 is set to be equal to the volume of the internal space of the recess 81 (volume of the recess 81). Thus, when the top-section-layer raw material liquid filling process at step S4 is completed, all recesses 81 are fully filled with the top-section-layer raw material liquid 91. However, the filling amount of the top-section-layer raw material liquid 91 may set to differ in accordance with the position of each recess 81 within the mold 80. For example, the filling amount of the top-section-layer raw material liquid 91 is set to be larger for the recess 81 closer to the center of the mold 80 and be smaller for the recess 81 closer to an end part of the mold 80, or the filling amount of the top-section-layer raw material liquid 91 is set to be larger for the recess 81 closer to the center of the mold 80 and be smaller for the recess 81 closer to an end part of the mold 80. The filling amount may be changed by changing, for example, the amount of one droplet, the number of droplets for each recess 81, or both the amount and number of droplets.

The relative movement of the nozzle 11a to each recess 81 is mainly performed on the XY coordinate of the XYZ stage 21, that is, in the in-plane direction of the surface 82 of the mold 80, but may involve movement in the Z-axis direction. For example, when the recesses 81 of the mold 80 have different sizes at different places, the nozzle 11a may be moved toward or away from the mold 80 to change the accuracy of landing.

When the top-section-layer raw material liquid filling process (step S4) is completed, the mold 80 is moved from the XYZ stage 21 to a wind dry unit (not illustrated) by, for example, a sterilized robotic arm. At the wind dry unit, for example, the filled mold 80 is sequentially placed on a belt conveyer (not illustrated) and moved through clean dry air. Then, at the end point of the belt conveyer, the mold 80 with the dried and solidified top-section-layer raw material liquid 91 is sequentially taken out and subjected to the following combination process.

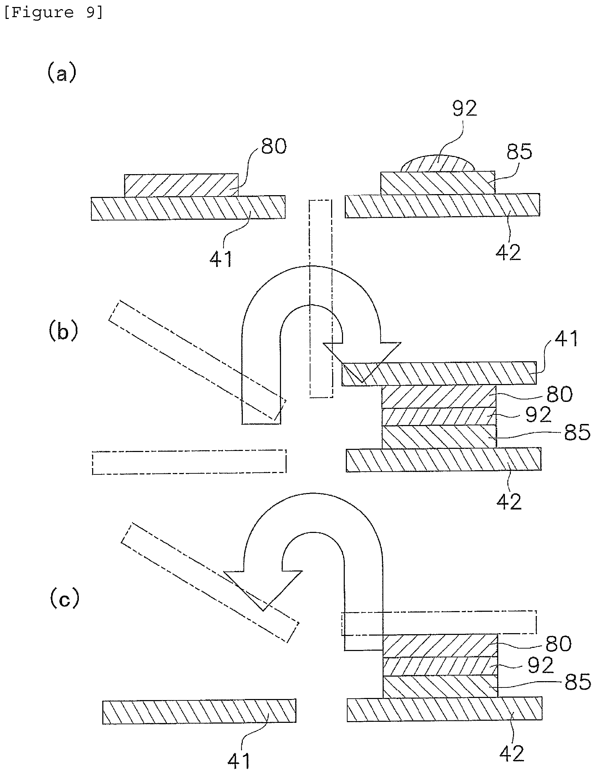

The operation at steps S11 to S15, which is performed in parallel with the operation at steps S1 to S5 described above, is performed by using a porous base member 85 (see Figure 9(a)). First, in a process of preparing the porous base member 85, surfaces of a predetermined number of the porous base members 85 are cleaned by, for example, air and then the porous base members 85 are arranged at a predetermined place. The prepared porous base members 85 are all sterilized through, for example, an autoclave (not illustrated) (step S12). Each sterilized porous base member 85 is sequentially subjected to positioning with respect to a dispenser (not illustrated) in a dispense unit by a feeder device (not illustrated) (step S13).

At step S14, bottom-section-layer raw material liquid 92 is distributed by the dispenser to the porous base members 85 and placed on the porous base member 85 in contact with the porous base member 85 as illustrated in Figure 9(a). The bottom-section-layer raw material liquid 92 is set to have, for example, a viscosity larger than 1 Pa•sec and smaller than 1000 Pa•sec, and the amount of the bottom-section-layer raw material liquid 92 is set to several tens mg for the mold 80 having a size of 20 mm × 20 mm. The bottom-section-layer raw material liquid 92 is a material that forms the lamination film 109 by a method to be described later, and thus preferably has a relatively high viscosity as described above. To settle the bottom-section-layer raw material liquid 92 having such a relatively high viscosity on the porous base member 85, the manufacturing method does not transition to a drying and bonding process (step S20) soon after the bottom-section-layer raw material liquid 92 is filled. The method includes a curing process (step S15) for allocating a time in which the bottom-section-layer raw material liquid 92 penetrates into the porous base member 85 by, for example, capillary action. The curing process only waits for an appropriate time of, for example, several seconds to several tens seconds. The curing process may involve, for example, a penetration promoting means that applies vibration or high pressure while the bottom-section-layer raw material liquid 92 is in contact with the porous base member 85.

In the subsequent combination process (step S20), the mold 80 is fixed on a suction stage 41 by suction as illustrated in Figure 9(a). The porous base member 85 is placed on a placement stage 42. To perform step S20, the mold 80 subjected to the drying process (step S5) is placed on the suction stage 41 by, for example, a sterilized robotic arm, and the porous base member 85 subjected to the curing process (step S15) is placed on the placement stage 42 by, for example, a sterilized robotic arm.

Subsequently, as illustrated in Figure 9(b), the suction stage 41 is moved up and inverted so that the mold 80 fixed to the suction stage 41 is placed over the porous base member 85 placed on the placement stage 42. While the mold 80 is placed over on the porous base member 85 as illustrated in Figure 9(b), the suction stage 41 is pressed toward the placement stage 42 to receive application of predetermined pressure. This predetermined pressure spreads the bottom-section-layer raw material liquid 92 sandwiched between the porous base member 85 and the mold 80. However, applied pressure is adjusted to the predetermined pressure to prevent the bottom-section-layer raw material liquid 92 from spreading out of the porous base member 85. To obtain such a result, for example, a preliminary experiment is performed to find an appropriate value of the predetermined pressure. The fixation of the mold 80 to the suction stage 41 may be maintained or canceled when the suction stage 41 applies pressure to the mold 80. Subsequently, as illustrated in Figure 9(c), the suction stage 41 is removed from the mold 80 while the fixation of the suction stage 41 to the mold 80 is canceled. The volume of the top-section-layer raw material liquid 91 decreases through drying, which leaves a step between the surface 82 of the mold 80 and the surface of a substance produced through solidification of the top-section-layer raw material liquid 91. The bottom-section-layer raw material liquid 92 enters into a space created inside each recess 81 due to this step, thereby forming the bottom-section layer 105 of the microneedles 103.

The mold 80 and the porous base member 85 in the state illustrated in Figure 9(c) is moved from the placement stage 42 to a stock unit (not illustrated). At the stock unit, the bottom-section-layer raw material liquid 92 between the mold 80 and the porous base member 85 is dried while a load is applied on the mold 80 from above. The load is applied on the mold 80 from above by, for example, a method of placing a weight on the mold 80 or a method of setting an assembly of the mold 80 and the porous base member 85 to a load stock dedicated machine configured to apply a load by air pressure or pressure through a spring.

### (6) Modifications

### (6-1) Modification 1A

The first embodiment describes the microneedles 103 having a two-layer structure in which the bottom-section layer 105 comprises no biologically active substance and provides no medical effect and the top-section layer 104 comprises a biologically active substance and provides a medical effect. For example, to extremely reduce a manufacturing error in the amount of drug comprised in the top-section layer 104, the amount of raw material liquid filling each recess needs to be extremely accurately controlled. In such a case, as compared to a conventional case in which the recess is filled with the raw material liquid by using a squeegee, the amount of the raw material liquid can be accurately controlled by the microneedle array manufacturing apparatus 1 and the microneedle array manufacturing method according to the first embodiment described above, in which the recess is filled with the raw material liquid in a predetermined number of droplets each having an adjusted fluid amount, and thus the amount of drug can be extremely accurately adjusted.

However, a microneedle array manufactured by the microneedle array manufacturing apparatus 1 and the microneedle array manufacturing method described in the first embodiment is not limited to the microneedle array 110 consisting of the microneedles 103 each having a two-layer structure described above. For example, the microneedle array manufacturing apparatus 1 and the microneedle array manufacturing method can manufacture a microneedle array consisting of microneedles each having a two-layer structure in which the top-section layer 104 comprises no biologically active substance and provides no medical effect and the bottom-section layer 105 comprises a biologically active substance and provides a medical effect. Alternatively, as in the above description of (4) Raw material liquid, the top-section layer and the bottom-section layer of each microneedle may both comprise biologically active medicinal substances. In addition, the microneedle array manufacturing apparatus 1 and the microneedle array manufacturing method can manufacture a microneedle array consisting of microneedles each having a multi-layer structure consisting of three or more layers. In this manner, the microneedle array manufacturing apparatus 1 and the microneedle array manufacturing method described in the first embodiment are suitable for manufacturing of a product including a microneedle array consisting of a plurality of microneedles each comprising a plurality of layers having compositions different from each other.

When a microneedle array is used in a field other than the medical field, for example, in fields related to beauty care and healthcare, the microneedles 103 may each have a two-layer structure in which the top-section layer 104 and the bottom-section layer 105 both comprise no biologically active substance and provide no medical effect.

At least one of the top-section layer 104 and the bottom-section layer 105 may be formed of biologically active substances without using polysaccharide exemplarily described in the first embodiment.

The top-section-layer raw material liquid described above may be, for example, solution of at least one or combination of aqueous polysaccharide, aqueous protein, polyvinyl alcohol, carboxy vinyl polymer, sodium polyacrylate described above. Examples of aqueous protein include serum albumin. The top-section-layer raw material liquid may comprise another substance such as monosaccharide or oligosaccharide. Examples of monosaccharide include glucose, and examples of oligosaccharide include disaccharide such as sucrose.

### (6-2) Modification 1B

In the above-described first embodiment, positioning is performed by capturing images of the alignment marks 83 through the CCD camera 22 and moving the XYZ stage 21 with reference to the alignment marks 83, but is not limited to such a method. For example, the positioning may be performed by pressing a side surface of the mold 80 to a jig to set a reference position.

### (6-3) Modification 1C

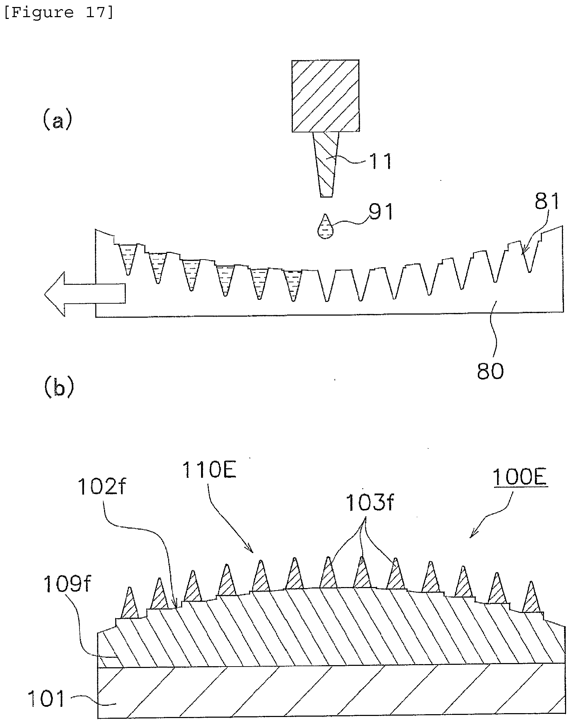

In the above-described first embodiment, the base member 101 has a flat plate shape, but may have a thin sheet shape or a three-dimensional shape with a curved surface. As illustrated in Figure 17(a), a surface 82A of a mold 80A may have a curved shape like a concave mirror, and the nozzle 11a may be moved along this curved surface of the surface 82 to fill each recess 81 with the top-section-layer raw material liquid 91. Accordingly, a plurality of microneedles 103f can be constantly formed in parallel to each other irrespective of a curved shape of a surface 102f of a fixing part 109f as illustrated in Figure 17(b). This configuration facilitates insertion of the microneedles 103f at any places of a microneedle array 110E. For example, when a microneedle array is formed on a sheet and the sheet is three-dimensionally deformed, microneedles are substantially vertical to a curved surface, but not in parallel to each other. This configuration makes difficult insertion of some of the microneedles and makes the microneedles prone to damage.

The fixing part 109f can be formed by a method same as that of the first embodiment by using the bottom-section-layer raw material liquid 92. The amount of composition comprised in each microneedle 103f can be accurately adjusted to an amount set in advance depending on the number of droplets of the top-section-layer raw material liquid 91.

The fixing part 109f may have any other three-dimensional shape. The above-described effect can be obtained when the microneedles 103f are three-dimensionally disposed on the surface 102f of the fixing part 109f and formed in parallel to each other.

### <Second embodiment>

### (7) Outline of microneedle array manufacturing method

In the above-described first embodiment, the droplet discharging apparatus 10 uses the single nozzle 11a, but in the second embodiment, as illustrated in Figure 10, the two nozzles 11a and 11b are used. The two cartridges 13a and 13b illustrated in Figure 10 contain top-section-layer raw material liquid 91 and 93 having different compositions.

The droplet discharging apparatus 10 illustrated in Figure 10 discharges, to the recesses 81 adjacent to each other, droplets of the top-section-layer raw material liquid 91 and droplets of the top-section-layer raw material liquid 93 alternately with the nozzle 11a and the nozzle 11b. The total amount of droplets discharged by the nozzles 11a and 11b differs between the recesses 81.

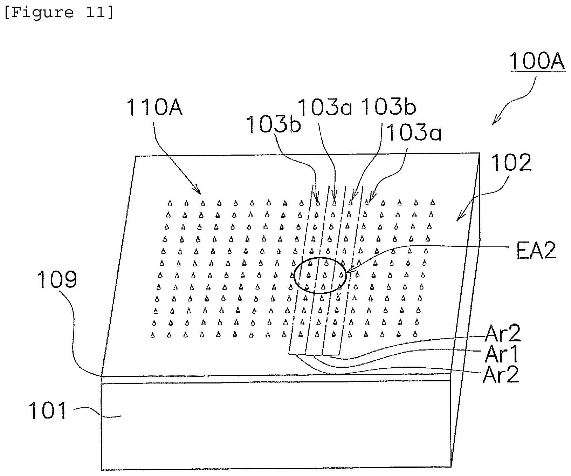

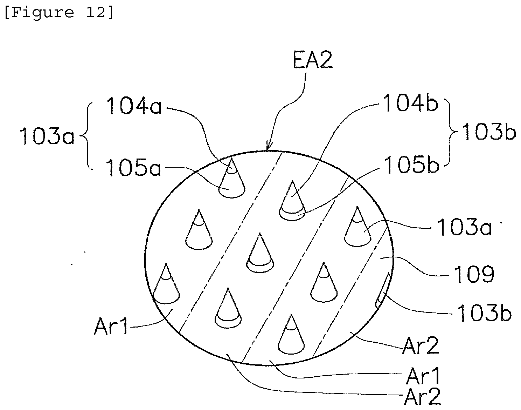

Figure 11 illustrates a product 100A including a microneedle array 110A formed by the above-described manufacturing method. Figure 12 illustrates a partial region EA2 of Figure 11 in an enlarged manner. Similarly to the microneedles 103 according to the first embodiment, a first microneedle 103a and a second microneedle 103b according to the second embodiment have two-layer structures consisting of top-section layers 104a and 104b at leading ends and bottom-section layers 105a and 105b next to the top-section layers 104a and 104b, respectively. In this example, the bottom-section layers 105a and 105b are second layers next to the top-section layers 104a and 104b. The top-section layer 104a and the bottom-section layer 105a of the first microneedle 103a are different from each other, and the top-section layer 104b and the bottom-section layer 105b of the second microneedle 103b are different from each other. The bottom-section layers 105a and 105b are formed of identical components by a method same as the first embodiment, but the top-section layers 104a and 104b have thickness different from each other, and thus the bottom-section layers 105a and 105b have thickness different from each other.

In this example, the kind of the composition (first composition) of the top-section layer 104a of the first microneedle 103a is different from the kind of the composition (third composition) of the bottom-section layer 105a of the first microneedle 103a, and the kind of the composition (second composition) of the top-section layer 104b of the second microneedle 103b is different from the kind of the composition (fourth composition) of the bottom-section layer 105b of the second microneedle 103b. The kind and amount of the composition (first composition) of the top-section layer 104a of the first microneedle 103a are different from the kind and amount of the composition (second composition) of the top-section layer 104b of the second microneedle 103b. In addition, the amount of the composition (third composition) of the bottom-section layer 105a of the first microneedle 103a is different from the amount of the composition (fourth composition) of the bottom-section layer 105b of the second microneedle 103b.

In Figures 11 and 12, a first area Ar1 is a line in which the first microneedle 103a is formed, and a second area Ar2 is a line in which the second microneedle 103b is formed. The first area Ar1 is sandwiched by the second areas Ar2 on both sides. Simultaneously, each second area Ar2 is sandwiched by the first areas Ar1 on both sides. In other words, a plurality of the first areas Ar1 and a plurality of the second areas Ar2 are alternately arranged.

With a conventional microneedle array manufacturing method using a squeegee, it is difficult to have microneedles in different structures alternately in lines adjacent to each other and accurately adjust the thicknesses of each layer in each microneedle, which can be, however, achieved by a method for manufacturing the microneedle array 110A according to the second embodiment.

As described above, in the method for manufacturing the microneedle array 110A according to the second embodiment, only the top-section-layer raw material liquid filling process (step S4) illustrated in Figure 6 is changed as described above, whereas the other processes are performed in a manner same as that of the first embodiment.

### (8) Modifications

### (8-1) Modification 2A

In the above-described second embodiment, the kind of the first composition is different from the kind of the third composition, and the kind of the second compositions different from the kind of the fourth composition. The kind and amount of the first composition are different from the kind and amount of the second composition. In addition, in the second embodiment, the amount of the third composition is different from the amount of the fourth composition. However, other combinations are possible as described below.

Specifically, the kind of the first composition is different from the kind of the third composition, the kind of the second composition is different from the kind of the fourth composition, the kind or amount of the first composition is different from the kind or amount of the second composition, and the kinds and amounts of the third composition and the fourth composition are different from each other.

For example, when the cartridge 13a and the cartridge 13b contain the top-section-layer raw material liquid 91 having the same composition, the first composition is different from the kind of the third composition, the second composition is different from the kind of the fourth composition, only the amounts of the first composition and the second composition are different from each other, and only the amounts of the third composition and the fourth composition are different from each other.

### (8-2) Modification 2B

The second embodiment describes lines of the first microneedles 103a arranged straight and the second microneedles 103b arranged straight as illustrated in Figures 11 and 12. However, the arrangement of microneedles in different structures is not limited to the arrangement illustrated in Figures 11 and 12. For example, m1 lines (m1 is a natural number) of the first microneedles 103a are arranged, n1 lines (n1 is a natural number) of the second microneedles 103b are arranged next, m2 lines (m2 is a natural number) of the first microneedles 103a are arranged next, and n2 lines (n2 is a natural number) of the second microneedles 103b are arranged next. In other words, the first microneedles 103a and the second microneedles 103b may be arranged alternately in an optional number of lines. A ratio of the total volume of the top-section layers 104a of the first microneedles 103a and the total volume of the top-section layer 104b of the second microneedle 103b comprised in one microneedle array is adjusted by adjusting each number of lines, thereby adjusting, for example, the administration amounts of two kinds of drugs.

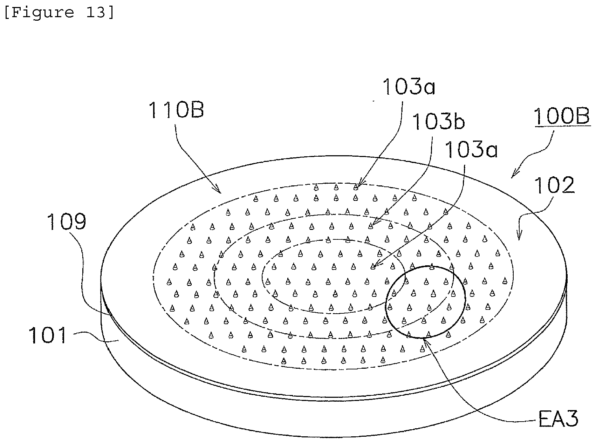

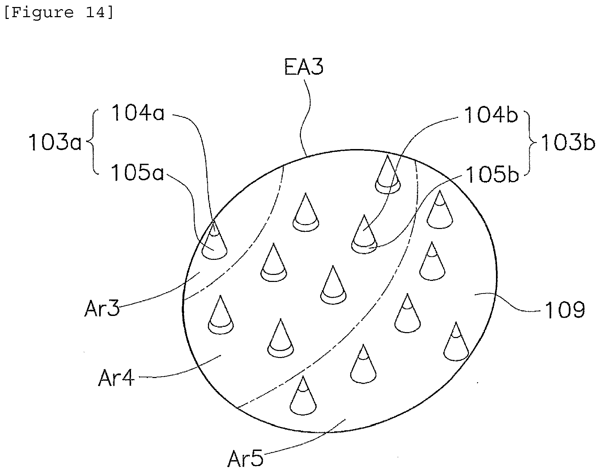

For example, as illustrated in Figures 13 and 14, lines of the first microneedles 103a may be arranged in a first area Ar3 having a circular shape and a first area Ar5 having a ring shape, and a second area Ar4 in which lines of the second microneedles 103b are arranged may be disposed surrounding the lines of the first microneedles 103a in the first area Ar3. In such a case, the first area Ar5 is disposed surrounding the second area Ar4. In this manner, when a microneedle array 110B is arranged in a circle and the microneedles 103a and 103b of different kinds are arranged in concentric circles, a situation that some microneedles of either kind are not in contact in use for, for example, drug administration is more likely to be avoided.

In this example, the microneedles 103a are same in the two first areas Ar3 and Ar5, but the kind (the kind and amount of a contained composition) of microneedles may be different between the two first areas Ar3 and Ar5, and the region denoted by Ar5 may be a third area. For example, in usage in the medical field, drug α of 20 wt% may be administered through microneedles in the first area Ar3, drug β of 35 wt% may be administered through microneedles in the second area Ar4, and drug γ of 45 wt% may be administered through microneedles in the third area.

### (8-3) Modification 2C

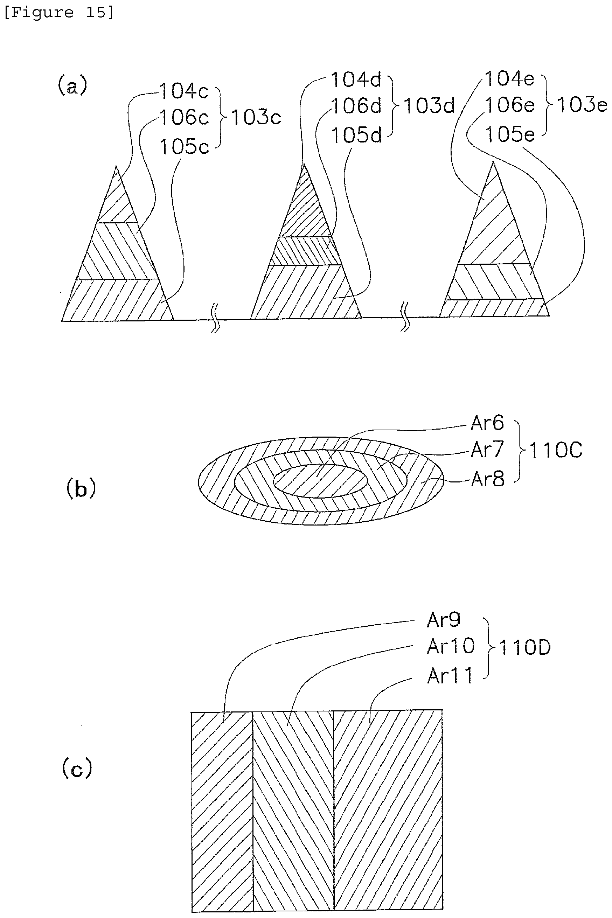

In the above-described first and second embodiments, the microneedles 103, 103a, and 103b have the two-layer structures in which the bottom-section layers 105, 105a, and 105b are formed next to the top-section layers 104, 104a, and 104b, respectively. However, each microneedle may have a structure of three layers or more. For example, like microneedles 103c, 103d, and 103e illustrated in Figure 15(a), intermediate layers 106c, 106d, and 106e may be formed between top-section layers 104c, 104d, and 104e and bottom-section layers 105c, 105d, and 105e, respectively. In this example, the intermediate layers 106c, 106d, and 106e are provided, but any number of a plurality of intermediate layers may be provided.

When such three-layer structures of the microneedles 103c, 103d, and 103e are formed, for example, a process of filling intermediate-layer raw material liquid and another drying process for drying the intermediate layer are added between the drying process (step S5) and the combination process (step S20) illustrated in Figure 6. In the intermediate-layer raw material liquid filling process, filling of the intermediate-layer raw material liquid can be achieved by using the droplet discharging apparatus 10 in a manner similar to the top-section-layer raw material liquid filling process. As illustrated in Figure 15(a), in manufacturing of a microneedle array having the microneedles 103c, 103d, and 103e of three kinds, the cartridge 13a or the cartridge 13b may be replaced after filling for the microneedles 103c and 103d of two kinds is finished by using the droplet discharging apparatus 10, and then filling for the microneedles 103e of another kind may be performed. However, when another droplet discharging apparatus (not illustrated) further including a third nozzle other than the nozzles 11a and 11b and a third cartridge other than the cartridges 13a and 13b is used, the replacement of a cartridge is not needed, thereby reducing a manufacturing time.

Similarly to the top-section-layer raw material liquid, the intermediate-layer raw material liquid is, for example, solution of a solid raw material in water, a mixed solvent of water and alcohol, or another solvent, or suspension liquid of a solid raw material in water, a mixed solvent of water and alcohol, or another solvent, or a mixture of the solution and the suspension liquid.

A microneedle array 110C may be provided that has a structure in which the microneedles 103c described above are arranged in a first area Ar6, the microneedles 103d are arranged in a second area Ar7, and the microneedles 103e are arranged in a third area Ar8 as illustrated in Figure 15(b). Alternatively, a microneedle array 110D may be provided that has a structure in which the microneedles 103c are arranged in a first area Ar9, the microneedles 103d are arranged in a second area Ar10, and the microneedles 103e are arranged in a third area Ar11, as illustrated in Figure 15(c).

In such a case, as for first microneedles 103c arranged in the first areas Ar6 and Ar9 and second microneedles 103d arranged in the second areas Ar7 and Ar10, the kind of the composition (first composition) of a top-section layer 104c of each first microneedle 103c may be different from the kind of the composition (third composition) of the intermediate layer 106c of the first microneedle 103c, and the kind of the composition (second composition) of the top-section layer 104d of each second microneedle 103d may be different from the kind of the composition (fourth composition) of the intermediate layer 106d of the second microneedle 103d. At least one of the kind and amount of the composition (first composition) of the top-section layer 104c of the first microneedle 103c may be different from the corresponding one of the kind and amount of the composition (second composition) of the top-section layer 104d of the second microneedle 103d. In addition, at least one of the kind and amount of the composition (third composition) of the intermediate layer 106c of the first microneedle 103c may be different from the corresponding one of the kind and amount of the composition (fourth composition) of the intermediate layer 106d of the second microneedle 103d. In this example, the intermediate layers 106c and 106d correspond to second layers.

Then, in usage in the medical field, a medicine can be produced that consists of a first drug α1 including a first component P1 in the top-section layer 104c and a second component P2 in the intermediate layer 106c in each first microneedle 103c, a second drug β1 including a third component Q1 in the top-section layer 104d and a fourth component Q2 in the intermediate layer 106d in each second microneedle 103d, and a third drug γ 1 including a fifth component R1 in the top-section layer 104e and a sixth component R2 in the intermediate layer 106e in each third microneedle 103e. In this medicine, for example, a drug administration amount can be adjusted by adjusting a ratio among the area of the first areas Ar6 and Ar9, the area of the second areas Ar7 and Ar10, and the area of the third areas Ar8 and Ar11 while the first microneedle 103c, the second microneedle 103d, and the third microneedle 103e are set to have identical weights and identical densities in the corresponding regions. For example, when the ratio among the area of the first areas Ar6 and Ar9, the area of the second areas Ar7 and Ar10, and the area of the third areas Ar8 and Ar11 is set to 4:7:9, a medicine comprising the drug α1 of 20 wt%, the drug β1 of 35 wt%, and the drug γ 1 of 45 wt% can be prepared.

### (8-4) Modification 2D

Although Figure 13 illustrates the case in which the first area Ar3 is concentrically comprised in the second area Ar4, the first area Ar3 may be comprised in the second area Ar4 in any other configuration. For example, a microneedle array may have a sea-island structure in which a plurality of first regions are scattered as islands in the sea of a second region.

### <Third embodiment>

### (9) Outline of apparatus for manufacturing a microneedle array and method for manufacturing the same

In the above-described first and second embodiments, the single nozzles 11a and 11b are used to filling for one microneedle. However, nozzles may be arrayed to perform the filling for one microneedle on a plurality of recesses 81 all at once.

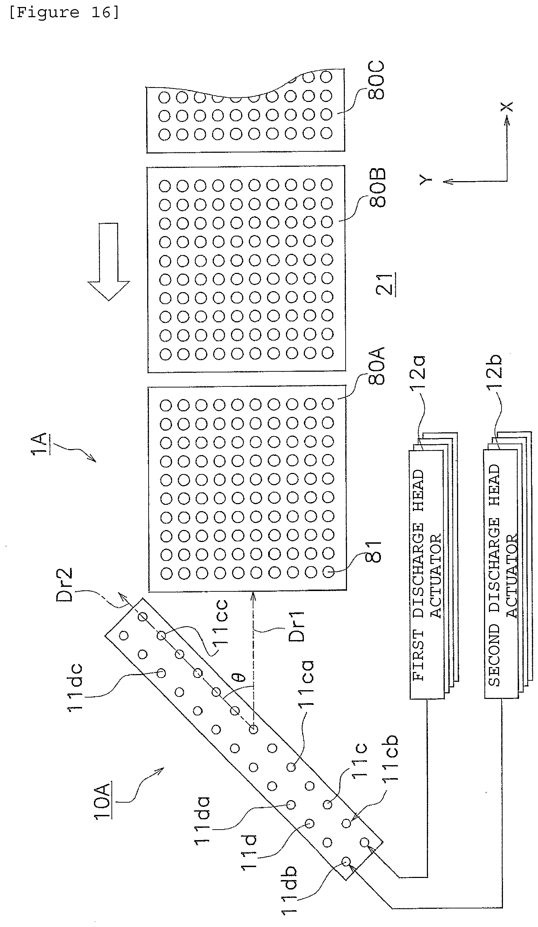

A droplet discharging apparatus 10A according to the third embodiment illustrated in Figure 16 includes arrayed nozzles 11c and 11d. The thirteen first discharge head actuators 12a are attached to the thirteen nozzles 11c, and the thirteen second discharge head actuators 12b are attached to the thirteen nozzles 11d.

Molds 80A, 80B, and 80C, which are continuously moved in the X-axis direction while being placed on the one XYZ stage 21, each include a matrix of the ten recesses 81 in the X-axis direction and the ten recesses 81 in the Y-axis direction.

As for each line of a plurality of the recesses 81 arranged parallel to the X axis, for example, droplets can be discharged into the recesses 81 in single lines through a nozzle 11ca and a nozzle 11da illustrated in Figure 16. This configuration of each single line is same as that of the droplet discharging apparatus 10 according to the second embodiment described with reference to Figure 10. Thus, the top-section-layer raw material liquid filling process (step S4) illustrated in Figure 6 can be performed on the recesses 81 in each line by the filling method in the second embodiment.

In this case, nozzles having no corresponding recesses 81 in the mold 80, for example, nozzles 11cb and 11db, are controlled not to discharge droplets by stopping operation of the corresponding first discharge head actuators 12a and stopping operation of the corresponding second discharge head actuators 12b.

Since the molds 80A, 80B, and 80C are continuously moved, the nozzle 11ca of one nozzle array discharges droplets to the mold 80A, while another nozzle 11cc discharges droplets to the next mold 80B. In this manner, when the droplet discharging apparatus 10A discharges droplets simultaneously to the plurality of sequential molds 80A and 80B, a time taken for filling by the droplet discharging apparatus 10A can be shortened.

In manufacturing of another lot, when droplets are discharged to molds between which the arrangement interval of the recesses 81 are different, the θ-axis stepping motor 21d illustrated in Figure 2 adjusts an angle θ between a direction Dr2 in which the nozzles 11c and 11d of the droplet discharging apparatus 10A are arranged and a relative moving direction Dr1 of the nozzles 11c and 11d. Accordingly, the Y coordinates of the plurality of nozzles 11c and 11d can be matched with the Y coordinates of the recesses 81.

An unillustrated configuration of a microneedle array manufacturing apparatus 1A in Figure 16 may be same as that of the microneedle array manufacturing apparatus 1 according to the first embodiment.

### (10) Modifications

### (10-1) Modification 3A

In the above-described third embodiment, the nozzles 11c and 11d are arranged in two lines, but the droplet discharging apparatus 10A may include nozzles in three lines or more. Alternatively, the droplet discharging apparatus 10A may only include the nozzles 11c arranged in one line.

### (10-2) Modification 3B

In the above-described third embodiment, the recesses 81 are arranged in a square shape. However, when the recesses 81 are arranged in a circular shape, droplets may be discharged while a mold is rotated in the direction of θ.

### (11) Characteristics

### (11-1)

As described above, the microneedle array manufacturing apparatuses 1 and 1A are configured to shape the microneedle arrays 110 and 110A to 110E consisting of the microneedles 103 and 103a to 103f by filling the recesses 81 formed in the molds 80, 80A, 80B, and 80C with raw material liquid for shaping the microneedles 103 and 103a to 103f, and include the droplet discharging apparatuses 10 and 10A and the positioning apparatus 20. As illustrated in, for example, Figure 8, the positioning apparatus 20 adjusts, through the XYZ stage 21 of the positioning apparatus 20, the relative positions of the nozzles 11a, 11b, 11c, and 11d of the droplet discharging apparatuses 10 and 10A and the mold 80 so that droplets land into each recess 81 from the droplet discharging apparatuses 10 and 10A. Then, as illustrated in, for example, Figure 8, the droplet discharging apparatuses 10 and 10A fill each recess 81 by discharging, to the recess 81, droplets 91a to 91e of the top-section-layer raw material liquid 91, each having a predetermined amount equal to or smaller than the volume of the recess 81.

In the microneedle array manufacturing method, the top-section-layer raw material liquid filling process (step S4) illustrated in Figure 6 is a first filling process of filling the recesses 81 with the top-section-layer raw material liquid 91 by landing, into the recesses 81 (exemplary first recesses) of the molds 80 and 80A to 80C, the droplets 91a to 91e of the top-section-layer raw material liquid 91 (exemplary first raw material liquid) in amounts equal to or smaller than the volumes of the recesses 81. The drying process (step S5) illustrated in Figure 6 is a drying process of drying the top-section-layer raw material liquid 91 in the recesses 81 to form the microneedle arrays 110 and 110A to 110E each consisting of the microneedles 103 and 103a to 103f.

In this manner, the amount of the top-section-layer raw material liquid 91 for each recess 81 is accurately adjusted by adjusting the total amount of the droplets 91a to 91e discharged to each recess 81. Since the concentration of any composition in the top-section-layer raw material liquid 91 is substantially uniform, the amount of any composition comprised in the top-section layers 104 and 104a to 104e formed by solidifying the top-section-layer raw material liquid 91 is accurately adjusted. As a result, the distribution of any composition of the microneedle arrays 110 and 110A to 110E is accurately adjusted.

### (11-2)

As described above, the droplet discharging apparatuses 10 and 10A are configured to discharge the droplets 91a to 91e as illustrated in Figure 8 in a total amount equal to or smaller than the volume of each recess 81. When the recess 81 having a circular cone shape as illustrated in Figure 8 is filled with one droplet of the top-section-layer raw material liquid 91, air bubbles are more likely to be included at a bottom part of the recess 81, and the top-section-layer raw material liquid 91 is more likely to spill out of the recess 81 due to bounce at landing. However, when the filling is performed with a plurality of droplets, less air bubbles are likely to be included at the bottom part, and the top-section-layer raw material liquid 91 is less likely to spill out of the recess 81, which facilitates accurate adjustment of the filling amount.

### (11-3)

Although each single recess 81 is filled with the five droplets 91a to 91e in the above-described case illustrated in Figure 8, the droplet discharging apparatuses 10 and 10A are configured to achieve such adjustment that the amount of one droplet at each discharge is equal to or smaller than one third of the volume of the recess 81. The droplet discharging apparatuses 10 and 10A and the positioning apparatus 20 are configured to achieve such positioning that three or more droplets land on different positions inside the recess 81. For example, in the case illustrated in Figure 8, the landing positions are landing points Lp1 to Lp5 different from each other. In this manner, when the droplets 91a to 91e land at different positions, air bubbles included in the recess 81 can be reduced and external spill of the top-section-layer raw material liquid 91 can be prevented for the top-section-layer raw material liquid 91 having a high viscosity.

### (11-4)

As described in the above-described second and third embodiments, when the cartridges 13a and 13b contain the top-section-layer raw material liquid 91 (exemplary first liquid) and the top-section-layer raw material liquid 93 (exemplary second liquid), the droplet discharging apparatuses 10 and 10A can separately discharge, as raw material liquid, the top-section-layer raw material liquid 91 and 93 having components different from each other by using the nozzle 11a and the nozzle 11b, or the nozzle 11c and the nozzle 11d. As a result, the various microneedles 103 and 103a to 103f can be combined to easily form the microneedle arrays 110 and 110A to 110E in various forms as illustrated in Figure 4, Figures 11 to 15, and Figure 17 for example.

### (11-5)

As described with reference to Figures 10 to 14, the droplet discharging apparatuses 10 and 10A and the positioning apparatus 20 are configured to fill the recesses 81 in the first areas Ar1, AR3, Ar5, Ar6, and Ar9 of the mold 80 with a first amount of raw material liquid, and fill the recesses 81 in the second area Ar2, AR4, Ar7, Ar6, and Ar10 of the mold 80 with a second amount of raw material liquid. As a result, the amount of composition in the first areas Ar1, AR3, Ar5, Ar6, and Ar9 and the amount of composition in the second area Ar2, AR4, Ar7, Ar6, and Ar10 can be accurately adjusted.

### (11-6)

Each recess 81 in which the top-section-layer raw material liquid 93 lands described with reference to Figure 10 can be regarded as a second recess. In this case, the top-section-layer raw material liquid filling process (step S4) in Figure 6 for filling the top-section-layer raw material liquid 93 is a second filling process of filling the recess 81 with the top-section-layer raw material liquid 93 by landing, in the recess 81 of the mold 80, droplets of the top-section-layer raw material liquid 93 (exemplary second raw material liquid) in an amount equal to or smaller than the volume of the recess 81. The drying process (step S5) in Figure 6 can be regarded as a drying process of drying the top-section-layer raw material liquid 93 in the recesses 81 to form the microneedle arrays 110A to 110E consisting of the microneedles 103a to 103f.

In this case, various kinds of microneedle arrays can be manufactured through various combinations of a region in which microneedles manufactured of the top-section-layer raw material liquid 91 (exemplary first raw material liquid) are arranged and a region in which microneedles manufactured of the top-section-layer raw material liquid 93 (exemplary second raw material liquid) are arranged.

As in the modification 2C described with reference to Figure 15, any recess 81 partially filled with the dried and solidified top-section-layer raw material liquid 91 and 93 (exemplary first raw material liquid) can be regarded as a second recess. Thus, the intermediate-layer raw material liquid filling process according to the modification 2C is a second filling process of filling the recess 81 with the intermediate-layer raw material liquid by landing, in a recess (exemplary second recess) of the mold 80, in which the top-section-layer raw material liquid 91 and 93 is dried, droplets of the intermediate-layer raw material liquid in an amount equal to or smaller than the volume of the recess 81 (in this case, a volume except for the volume of the dried and solidified top-section-layer raw material liquid 91 and 93). The intermediate-layer raw material liquid drying process can be regarded as a drying process of drying the intermediate-layer raw material liquid in the recesses 81 to form the microneedle arrays 110A to 110E consisting of the microneedles 103a to 103f.

In this case, various kinds of the microneedle arrays 110C and 110D can be manufactured through various combinations of the top-section layers 104c, 104d, and 104e manufactured of the top-section-layer raw material liquid 91 and 93 (exemplary first raw material liquid) and the intermediate layers 106c, 106d, and 106e manufactured of the intermediate-layer raw material liquid (exemplary second raw material liquid).

### (11-7)

The combination process (step S20) and the drying and bonding process (step S21) in Figure 6 are a fixation process of fixing the microneedles 103a to 103f comprising parts shaped with the dried top-section-layer raw material liquid 91 and 93 onto the porous base member 85 by placing the porous base member 85, at least part of a surface of which is covered by the bottom-section-layer raw material liquid 92 (exemplary third raw material liquid), over a surface of the mold 80, on which the recesses 81 are formed, and drying the bottom-section-layer raw material liquid 92.

Since at least part of the surface of the porous base member 85 is covered by the bottom-section-layer raw material liquid 92, the bottom-section-layer raw material liquid 92 is more likely to penetrate into pores of the porous base member 85, which facilitates formation of the products 100 and 100A having microneedles firmly fixed to the porous base member 85. This effect improves when the curing process (step S15) is provided before the fixation process.

### (11-8)

For example, as described with reference to Figures 11 to 15, the first microneedles 103a and 103c are formed in at least one of the first areas Ar1, Ar3, Ar5, Ar6, and Ar9, and comprise the first composition in the top-section layers 104a and 104c at the leading ends thereof, and the third composition in the bottom-section layer 105a (exemplary second layer of the first microneedle) or the intermediate layer 106c (exemplary second layer of the first microneedle) provided next. The second microneedles 103b and 103d are formed in at least one of the second areas Ar2, Ar4, Ar7, and Ar10 adjacent to the first areas Ar1, Ar3, Ar5, Ar6, and Ar9, and comprise the second composition in the top-section layers 104b and 104d at the leading ends thereof and the fourth composition in the bottom-section layer 105b (exemplary second layer of the second microneedle) or the intermediate layer 106d (exemplary second layer of the second microneedle) provided next. When the above-described microneedle array manufacturing apparatus or microneedle array manufacturing method is used, the kind of the first composition is different from the kind of the third composition, the kind of the second composition is different from the kind of the fourth composition, at least one of the kind and amount of the first composition is different from the corresponding one of the kind and amount of the second composition, and at least one of the kind and amount of the third composition is different from the corresponding one of the kind and amount of the fourth composition.

As a result, when products 100, 100A, 100C, and 100D including microneedle arrays are used in, for example, the medical field, various drugs can be prepared and the products can be used in various situations of drug administration.

### (11-9)

When the above-described microneedle array manufacturing apparatus or microneedle array manufacturing method is used, at least one of the second areas Ar2, AR4, and Ar7 can be disposed surrounding at least one of the first areas Ar1, AR3, and Ar6. As a result, the products 100, 100A, and 100C including microneedle arrays can prevent failure of usage of only microneedles in one of the areas when not all microneedles contact skin, for example.

### (11-10)

As described above in the modification 1C with reference to Figures 17(a) and 17(b), a product 100E including a microneedle array includes the fixing part 109f and the microneedles 103f. The surface 102f of the fixing part 109f has a three-dimensional shape curved like a concave mirror. Thus, the microneedles 103f are three-dimensionally arranged on the surface 102f of the fixing part 109f along the three-dimensional shape curved like a concave mirror. Moreover, since the microneedles 103f are formed in parallel to each other, all microneedles 103f extend in a pressing direction, which facilitates insertion. This configuration also facilitates attachment to a relatively small place such as an ear having a complicated three-dimensional shape.

### <Fourth embodiment>

### (12) Method for producing intermediate layer for producing microneedle patch having excellent breaking property

A microneedle is largely divided into a top section comprising drug and a bottom-section layer comprising no drug. The present invention provides a microneedle producing method in which, in production of the microneedle, the top section is first produced and then a thin layer (intermediate layer) is produced by using raw material that has a strength clearly different from that of raw material for producing the other two layers and has a high breaking property, instead of spraying the raw material for producing the bottom-section layer immediately after the drying process is completed, thereby enabling restriction of a damaged part of the microneedle to this fragile boundary. Thus, when the intermediate layer is produced at an end of a top section as a drug containing part, which is closer to the bottom-section layer, the top section containing drug breaks at a basis-side stump and is left in a target dermis, thereby achieving administration of the entire amount of this drug. The strength of raw material for producing this intermediate part, after drying, is set to be clearly different from those of the other two parts (the top section and the bottom-section layer). However, when two or more top-section layers comprise drug, this intermediate part is set between a part that breaks and is left in skin epidermis and dermis and a bottom-section layer. Similarly, in a microneedle including a larger number of top-section layers containing drug, an intermediate part is formed between a part that breaks and is left in skin and a bottom-section layer. However, the position of this intermediate layer is not limited to the position described in the previous section because of the characteristic of drug contained in the microneedle and an administration purpose.

### (13) Production of two-layer intermediate layer

The intermediate layer produced in the previous section has a single-layer structure and a strength clearly different from those of other sites, and is specialized to provoke reliable breaking. Instead, an intermediate layer (first intermediate layer) produced after a top section containing drug is manufactured and hardened by drying is manufactured and hardened by drying, and then the same operation is repeated to separately form, manufacture, and hardened by drying an intermediate layer (second intermediate layer). The ratio of the thicknesses of the intermediate layers may be 1:1 but may be optionally set as long as the thicknesses satisfy thicknesses to be described later.

### (14) Thickness of intermediate layer

When having a single-layer structure, an intermediate layer of a microneedle in the previous section may have a thickness of 5 µm to 50 µm, preferably a thickness of 10 µm to 30 µm, more preferably a thickness of 15 µm to 20 µm. When the second intermediate layer is included, thicknesses substantially twice as large as the thicknesses listed above are applicable. Specifically, when two intermediate layers of a microneedle are included, the intermediate layers may have an entire thickness of 10 µm to 100 µm, preferably an entire thickness of 20 µm to 60 µm, more preferably an entire thickness of 30 µm to 40 µm. After a layer comprising a biologically active material (drug) is produced and dried, this intermediate layer is formed by adjusting the raw material to have a plane surface and dried, and then a basis layer is produced. Through the present process, a microneedle acquires capability of reliably breaking at a specified position to be reliably left in a short time and can be formed to have a function to prevent backflow of, through a penetrating hole, drug released from the top section into dermis, when a raw material having a dissolution speed slower than that of a top section is selected as a raw material for producing the first intermediate layer.

### (15) Microneedle strength setting

The following describes the strength of each component of a microneedle according to the present invention in an example with a microneedle containing one kind of drug and including a bottom-section layer comprising no drug, and describes setting of the strength of each layer. The strength of a microneedle produced in the present invention is highest in the bottom-section layer, followed in order by a top section comprising the drug, and an intermediate layer. However, when the top section as a drug containing part includes two or more layers, an intermediate part is set between a part that breaks and is left in skin and the bottom-section layer, the strength is also highest in the bottom-section layer, followed in order by the top section comprising the drug, and the intermediate layer. To achieve this purpose, the weight-average molecular weight of a polymer compound included in each layer is highest in the top section, followed by the bottom-section layer, and can be set further lower in the intermediate part. However, the weight-average molecular weight for each layer may be the same or in the inverse order when a strength condition is satisfied. Alternatively, the hardness and weight-average molecular weight of the intermediate layer may be highest. When an intermediate layer including two layers is produced, the first intermediate layer is formed of a polymer material having a high hardness and a low absorbability and selected as a raw material set to have high concentration, and the second intermediate layer has a strength clearly weaker than those of the first intermediate layer and the bottom-section layer, has a high absorbability, and is formed of a polymer material selected as a raw material set to low concentration. However, setting of the strengths of the intermediate layers or the like is not limited to the above-described relation because of, for example, difference in used drug, and only one of the intermediate layers may be formed. To further efficiently achieve this process, a larger number of intermediate layers may be formed, and drug may or may not be contained in any of the intermediate layers. Instead of producing the intermediate layers right after the top section as a drug containing part is produced, the intermediate layers may be produced after part of the bottom-section-layer raw material is discharged and hardened, and then the rest of the bottom-section layer raw material may be discharged to complete formation of the entire bottom-section layer. Each intermediate layer of a microneedle produced in this manner is formed not at a joint surface of the top section and lower-layer sections but in the bottom-section layer. Any kind of drug may be added. In this case, it is essential to include a plurality of droplet discharging apparatuses each capable of discharging raw material obtained by changing, as appropriate, the mixture ratio and concentration of a bioabsorbable formulation optimized for forming a microneedle produced by the microneedle array manufacturing apparatus according to the present invention.

### (16) Production of basis layer integrated with substrate including contact surface

A microneedle array forming method in which, when produced by an inkjet method, a microneedle array is formed integrally with a bonding surface and a flat surface adhering to a substrate when part or all of the bottom-section layer is produced, whereby collapse of microneedles due to piercing is prevented and a top section containing drug easily and reliably breaks from the bottom-section layer. In this case, the bonding surface of the bottom-section layer with the top section may be flat, but does not necessarily limited thereto. In such a case, when the bottom-section layer is formed after an intermediate layer in the previous section is formed by spraying the raw material, a microneedle can be formed to be capable of more reliably breaking at the intermediate layer to leave the top section. In this method, too, the intermediate layer may have a two-layer structure or a multi-layer structure as described in the previous section.

### (17) Contribution of intermediate layer to reduction of treatment time

In treatment with a microneedle patch produced by a conventional method and thus including no intermediate layer, in order to reliably perform drug administration, a microneedle patch needs to be fixed to an administration site for 10 minutes to 30 minutes at minimum to one hour to two hours at maximum in accordance with a dissolution time of the raw material of the top section as a drug containing part, until the drug containing part dissolves and drug administration is completed. However, with a microneedle patch including an intermediate layer having an excellent breaking property, the top section containing drug is instantaneously separated at the intermediate part in dermis substantially simultaneously with insertion and left in the dermis, and thus the bottom-section layer can be removed right after the top section is separated irrespective of the dissolution time of a polymer raw material of the top section as a drug containing part. Accordingly, the series of the drug administration of the microneedle patch is completed within 5 seconds to 20 seconds approximately.

### (18) Prevention of backflow of drug dissolved and released in dermis through penetrating hole through which microneedle is inserted

When a microneedle produced in the previous section invention is inserted into skin to allow the top section containing drug to reach at a target site inside the dermis, a formed polymer compound spontaneously dissolves to release the contained drug in the dermis. In this case, since an intermediate layer having a weak strength and a high absorbability is provided at a basis side of the top section containing the drug, backflow through the penetrating hole is prevented to certain extent. In such a case, when the first intermediate layer in the previous section is produced in addition, the first intermediate layer is formed of a polymer material having a high hardness and a low absorbability and selected as the raw material set to have high concentration, and thus provides an effect of preventing the backflow by completely closing the penetrating hole for a certain duration after the polymer compound forming the top section is dissolved to release the drug. This complete closure duration may be freely set to a desired duration by controlling a dissolution property of the polymer material used in the formation. The dissolution time of this layer is 10 minutes to 24 hours, preferably 15 minutes to 6 hours, more preferably 30 minutes to 3 hours. The present invention is not limited to these time durations but may be any duration as long as the entire amount of the drug contained in the top section and administered is reliably dissolved and released at the target site inside the dermis.

### (19) Description of the invention with reference to drawings

### (19-1) Method for manufacturing microneedle patch

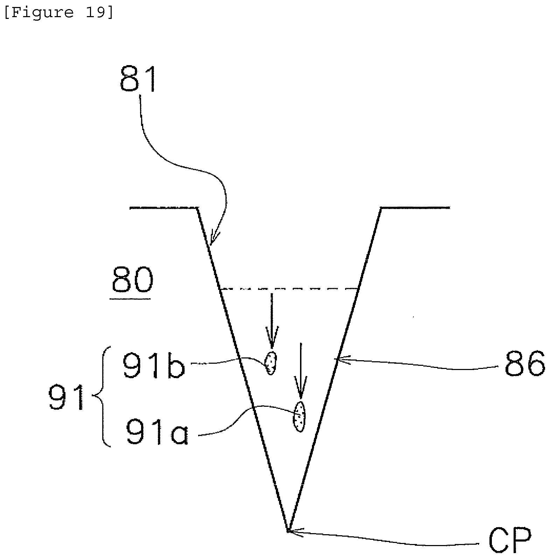

The following describes, with reference to Figures 19 to 22, a method for manufacturing a microneedle patch having a plurality of microneedles each including the top-section layer 104 (corresponding to the top section as a drug containing part described above) to be described later, an intermediate layer 106 (corresponding to a thin layer having a high breaking property described above), and the bottom-section layer 105 (corresponding to the bottom-section layer described above).

Figure 19 illustrates a state in which droplets 91a and 91b for filling with the top-section-layer raw material liquid 91 (first raw material liquid) comprising drug are discharged into a top-section layer formation site 86 of each recess 81 of the mold 80 by using the microneedle array manufacturing apparatuses 1 and 1B described above.

After the top-section layer formation site 86 from a central part CP to a height illustrated with a dashed line in Figure 19 is filled with the top-section-layer raw material liquid 91 (first raw material liquid), the top-section-layer raw material liquid 91 (first raw material liquid) is hardened by drying. The hardening by drying means solidification.

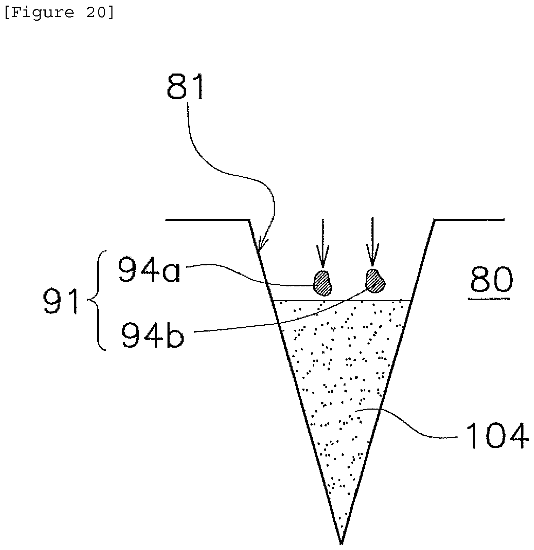

Figure 20 illustrates a state in which droplets 94a and 94b for filling with the intermediate-layer raw material liquid 94 (second raw material liquid) are discharged onto the hardened top-section layer 104 by using the above-described microneedle array manufacturing apparatuses 1 and 1B.

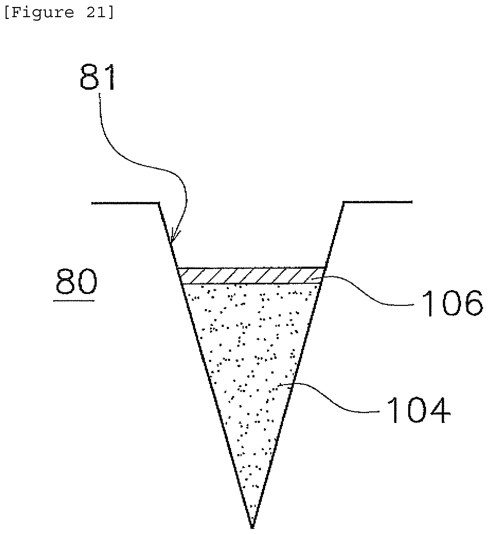

Figure 21 illustrates a state in which the intermediate-layer raw material liquid 94 (second raw material liquid) is hardened by drying to form the intermediate layer 106 on the top-section layer 104 in contact. When the intermediate layer 106 is formed, the intermediate-layer raw material liquid 94 (second raw material liquid) is dried for a slightly longer time.

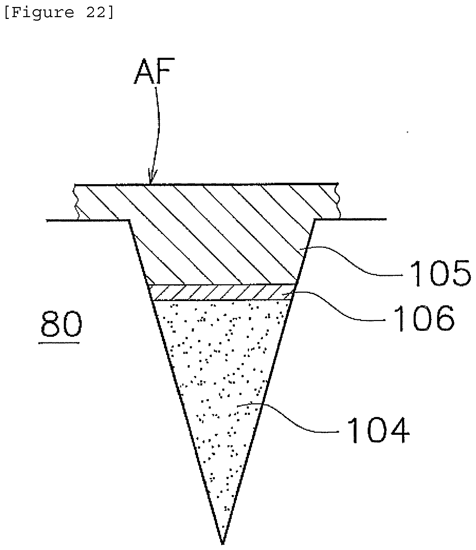

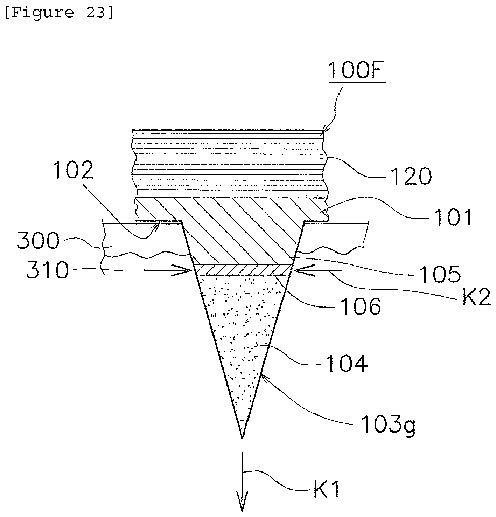

Figure 22 illustrates a state in which the bottom-section layer 105 is formed on the intermediate layer 106, while being in contact with the intermediate layer 106. The bottom-section layer 105 is formed through filling with the bottom-section-layer raw material liquid 92 (third raw material liquid) by using the microneedle array manufacturing apparatuses 1 and 1B. An upper surface of the bottom-section layer 105, which is opposite the intermediate layer 106, is a bonding surface AF onto which a support member 120 illustrated in Figure 23 is to be bonded.

### (19-2) Method of using microneedle patch

The following describes, with reference to Figures 23 to 26, a method of using the above-described microneedle patch having a plurality of microneedles each including the top-section layer 104, the intermediate layer 106, and the bottom-section layer 105.

Figure 23 illustrates a microneedle 103g inserted into a dermis 310 through an epidermis 300. The microneedle 103g includes the top-section layer 104, the intermediate layer 106, and the bottom-section layer 105 described above with reference to Figures 19 to 22. A microneedle patch 100F having a plurality of the microneedles 103g and the support member 120 is a product including a microneedle array.

As illustrated in Figure 23, when the surface 102 of the base member 101 contacts the epidermis 300, the bottom-section layer 105 penetrates through the epidermis 300, and the intermediate layer 106 and the top-section layer 104 reach at the dermis 310. In Figure 23, arrow K1 indicates a direction in which the microneedle 103g is inserted. Arrow K2 indicates a place where the microneedle 103g breaks.

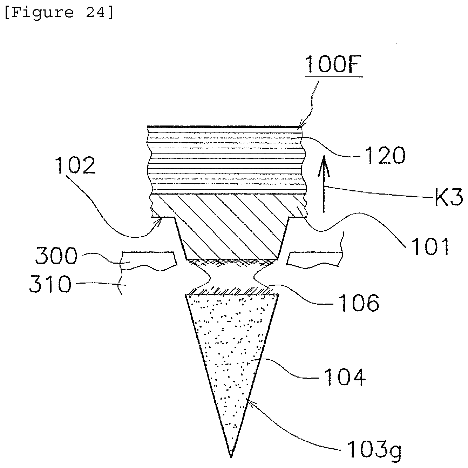

Figure 24 illustrates a state in which the intermediate layer 106 of the microneedle 103g inserted into the dermis 310 is broken. In Figure 24, arrow K3 indicates a direction in which the microneedle patch 100F is removed from skin. When the microneedle patch 100F is removed, the intermediate layer 106 having a weakest breaking strength in the microneedle 103g breaks, and the top-section layer 104 is left in the dermis 310.

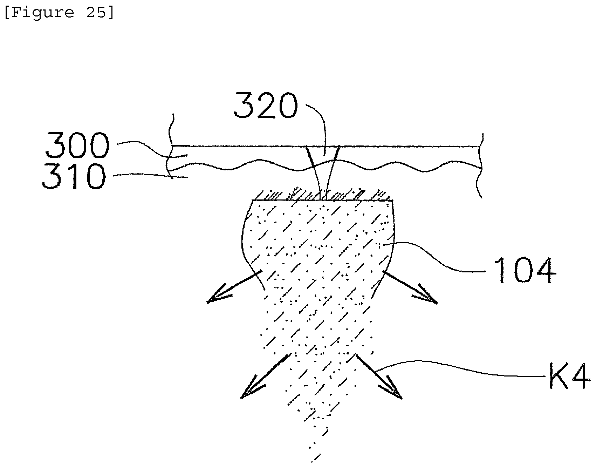

Figure 25 illustrates dissolution of the top-section layer 104 as a drug containing part containing drug and release of the drug. After the microneedle patch 100F is removed from the skin, an extremely small insertion hole 320 is formed on the epidermis 300 as illustrated in Figure 25. In Figure 25, arrow K4 represents release of the drug contained in the top-section layer 104.

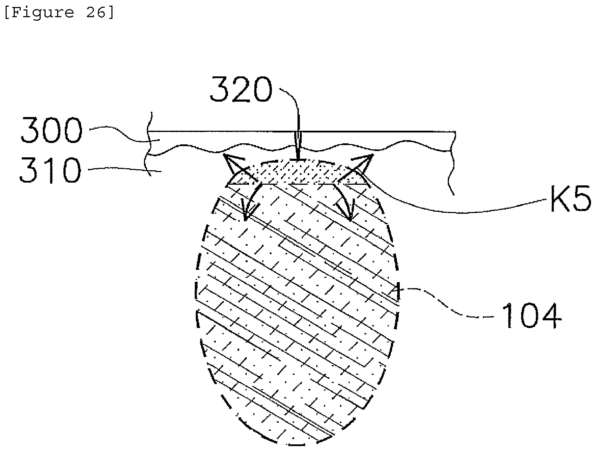

Figure 26 illustrates a state in which the intermediate layer 106 dissolves following the top-section layer 104. In Figure 26, arrow K5 represents dissolution of the intermediate layer 106. In Figure 26, a range enclosed by a dashed line represents a range in which the dissolved top-section layer 104 diffuses.

### (19-3) Another method for manufacturing microneedle patch

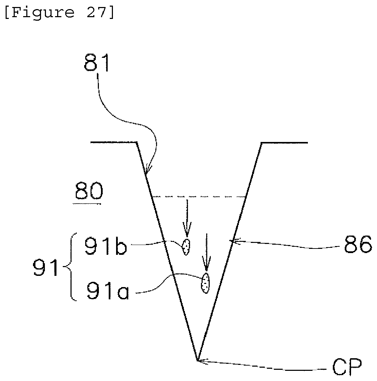

The following describes, with reference to Figures 27 to 30, a method for manufacturing a microneedle patch having a plurality of microneedles each including the top-section layer 104 (corresponding to a top section as a drug containing part described above) to be described later, a first intermediate layer 1061, a second intermediate layer 1062, and the bottom-section layer 105 (corresponding to a bottom-section layer described above).

Figure 27 illustrates a state in which droplets 91a and 91b for filling with the top-section-layer raw material liquid 91 (first raw material liquid) comprising drug are discharged at the top-section layer formation site 86 of each recess 81 of the mold 80 by using the above-described microneedle array manufacturing apparatuses 1 and 1B.

After the top-section layer formation site 86 from the central part CP to a height illustrated with a dashed line in Figure 27 is filled with the top-section-layer raw material liquid 91 (first raw material liquid), the top-section-layer raw material liquid 91 (first raw material liquid) is hardened by drying.

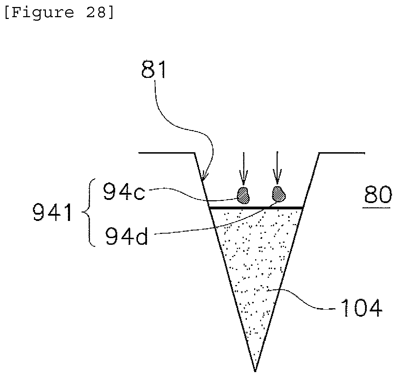

Figure 28 illustrates a state in which droplets 94c and 94d for filling with first intermediate-layer raw material liquid 941 (second raw material liquid) are discharged onto the hardened top-section layer 104, by using the above-described microneedle array manufacturing apparatuses 1 and 1B.

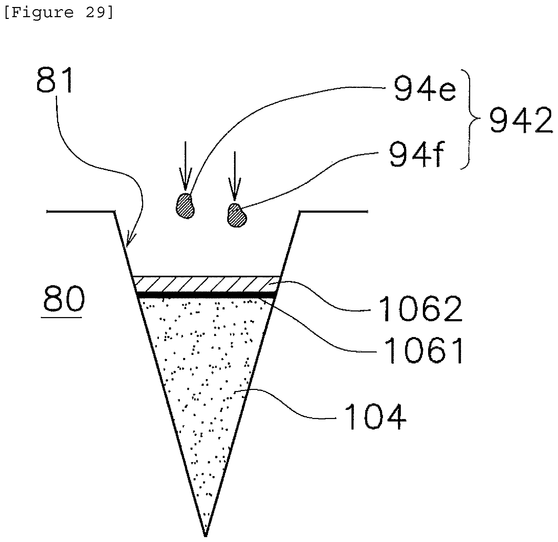

Figure 29 illustrates a state in which the first intermediate-layer raw material liquid 941 (second raw material liquid) is hardened by drying to form the first intermediate layer 1061 on the top-section layer 104 in contact. Figure 29 illustrates a state in which droplets 94e and 94f for filling with second intermediate-layer raw material liquid 942 are discharged onto the first intermediate layer 1061 by using the above-described microneedle array manufacturing apparatuses 1 and 1B. When the second intermediate layer 1062 is formed, the second intermediate-layer raw material liquid 942 (third raw material liquid) is dried for a slightly longer time.

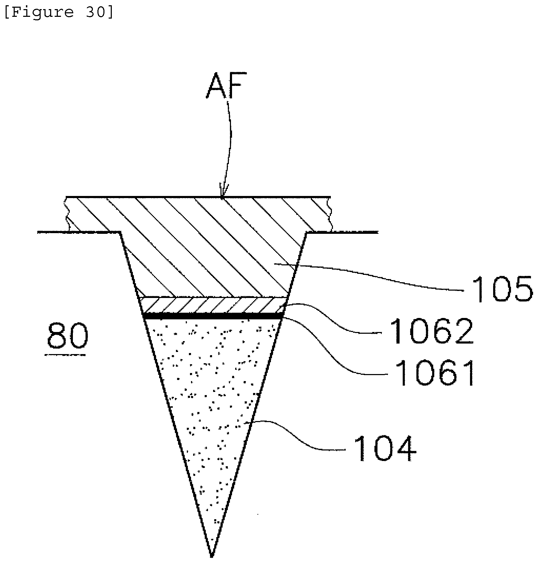

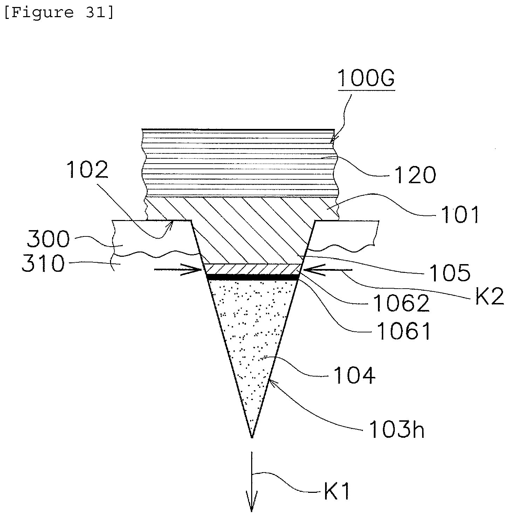

Figure 30 illustrates a state in which the bottom-section layer 105 is formed on the second intermediate layer 1062 while being in contact with the second intermediate layer 1062. The bottom-section layer 105 is formed through filling with the bottom-section-layer raw material liquid 92 (fourth raw material liquid) by using the microneedle array manufacturing apparatuses 1 and 1B. An upper surface of the bottom-section layer 105, which is opposite the second intermediate layer 1062, is a bonding surface AF to which the support member 120 illustrated in Figure 31 is to be bonded.

### (19-4) Another method of using microneedle patch

The following describes, with reference to Figures 31 to 34, a method of using the above-described microneedle patch having a plurality of microneedles each including the top-section layer 104, the first intermediate layer 1061, the second intermediate layer 1062, and the bottom-section layer 105.

Figure 31 illustrates a microneedle 103h inserted into dermis 310 through epidermis 300. The microneedle 103h includes the top-section layer 104, the first intermediate layer 1061, the second intermediate layer 1062, and the bottom-section layer 105 described above with reference to Figures 27 to 30. A microneedle patch 100G having a plurality of the microneedles 103h and the support member 120 is a product including a microneedle array.

As illustrated in Figure 31, when the surface 102 of the base member 101 contacts the epidermis 300, the bottom-section layer 105 penetrates through the epidermis 300, and the first intermediate layer 1061, the second intermediate layer 1062, and the top-section layer 104 reach at the dermis 310. In Figure 31, arrow K1 indicates a direction in which the microneedle 103h is inserted. Arrow K2 indicates a place at which the microneedle 103h breaks.

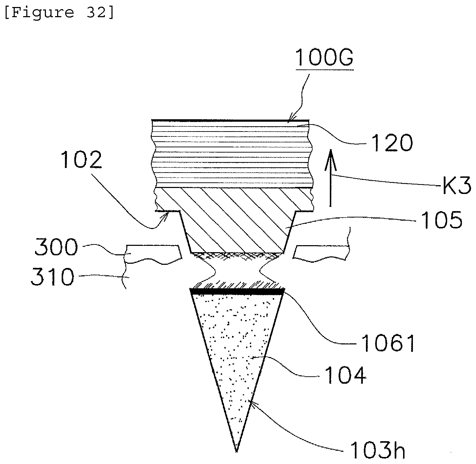

Figure 32 illustrates a state in which the second intermediate layer 1062 of the microneedle 103h inserted into the dermis 310 is broken. In Figure 32, arrow K3 indicates a direction in which the microneedle patch 100G is removed from skin. When the microneedle patch 100G is removed, the second intermediate layer 1062 having a weakest breaking strength in the microneedle 103h breaks, and the top-section layer 104 is left in the dermis 310.

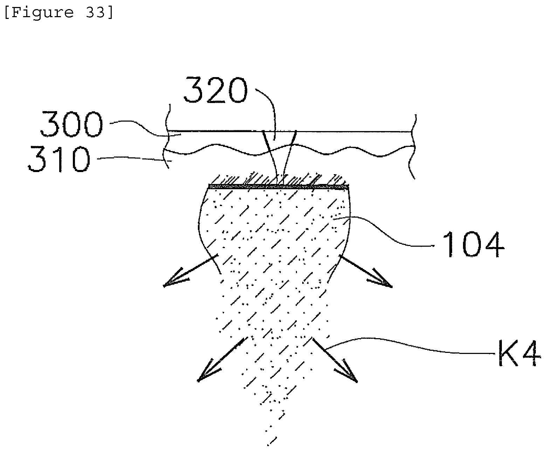

Figure 33 illustrates dissolution of the top-section layer 104 as a drug containing part containing drug and release of the drug. After the microneedle patch 100G is removed from the skin, an extremely small insertion hole 320 is formed in the epidermis 300 as illustrated in Figure 33. In Figure 33, arrow K4 represents release of the drug contained in the top-section layer 104.

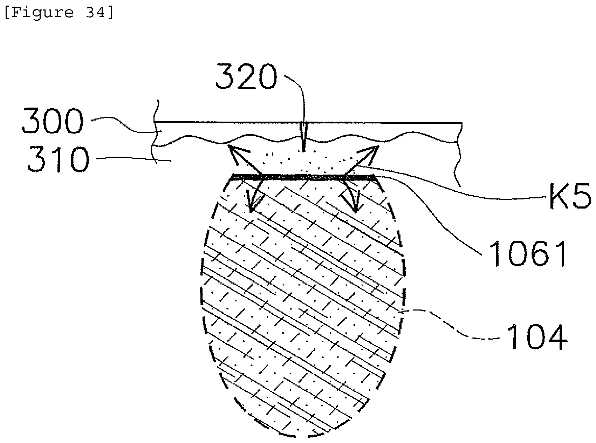

Figure 34 illustrates a state in which the intermediate layer 106 dissolves following the top-section layer 104. In Figure 34, arrow K5 represents dissolution of the first intermediate layer 1061. In Figure 34, a range enclosed by a dashed line represents a range in which the dissolved top-section layer 104 diffuses.

### (20) Remarks

### (20-1) Prior art of invention

### (Name of prior art)

### MICRONEEDLE ARRAY MANUFACTURING APPARATUS, MICRONEEDLE ARRAY MANUFACTURING METHOD, AND PRODUCT INCLUDING MICRONEEDLE ARRAY

### (Technical field)

This prior art relates to a microneedle array manufacturing apparatus and a microneedle array manufacturing method for manufacturing a microneedle array consisting of a plurality of microneedles, and a product including the microneedle array.

### (Problem solved by prior art)

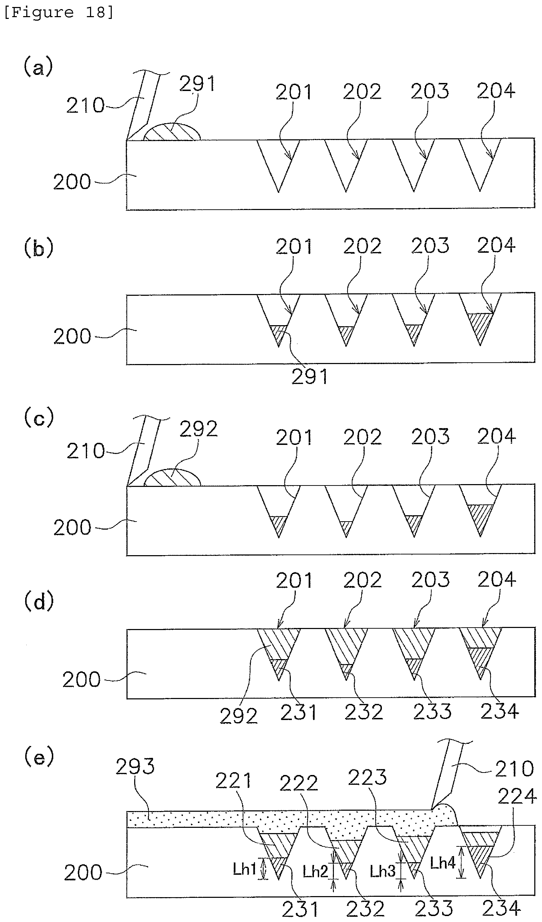

In a method of alternately repeating filling with a needle raw material and drying thereof in the order of Figure 18(a), Figure 18(b), Figure 18(c), Figure 18(d), and Figure 18(e) by using a stamper 200 as a kind of mold disclosed in patent document 1 and a squeegee 210, the filling amounts of microneedle raw materials 291, 292, and 293 filling recesses 201, 202, 203, and 204 tend to have large error. For example, difference is generated between the thicknesses Lh1, Lh2, Lh3, and Lh4 of upper layers 231, 232, 233, and 234 of microneedles 221, 222, 223, and 224. The difference in these layer thicknesses degrades the accuracy of composition distribution, and accordingly, the amount of drug differs between the four microneedles 221, 222, 223, and 224, or large error occurs in the amount of drug as a whole. In particular, production of a thin uniform plane layer such as an intermediate layer according to the present invention can be optimally performed by the microneedle array manufacturing apparatuses 1 and 1A described above, and a highly useful product can be provided by the microneedle manufacturing apparatus according to the present invention.

The prior art is intended to provide a microneedle array manufacturing apparatus and a microneedle array manufacturing method capable of accurately adjusting composition distribution of a microneedle array and provide a product including the microneedle array having accurately adjusted composition distribution.

### (Means for solving problem in another expression)

A microneedle patch according to a first aspect includes a base member and a plurality of microneedles supported by the base member. Each microneedle includes a top-section layer comprising a biologically active substance to be pierced into dermis, and an intermediate layer provided between the top-section layer and the base member, including composition having breaking strength weaker than breaking strength of composition of the top-section layer, and having a thickness of 5 µm to 100 µm inclusive.

A microneedle patch according to a second aspect is the microneedle patch according to the first aspect in which the intermediate layer has a thickness of 10 µm to 30 µm inclusive.

A microneedle patch according to a third aspect is the microneedle patch according to the second aspect in which the intermediate layer has a thickness of 15 µm to 20 µm inclusive.

A microneedle patch according to a fourth aspect is the microneedle patch according to any one of the first to third aspects in which the intermediate layer is made of a material that dissolves in dermis following the top-section layer.

A microneedle patch according to a fifth aspect is the microneedle patch according to any one of the first to fourth aspects in which the intermediate layer is adjusted to break in dermis in 20 seconds or less.

A microneedle patch according to a sixth aspect is the microneedle patch according to the fifth aspect in which the intermediate layer is adjusted to break in dermis in 5 seconds or less.

A microneedle patch according to a seventh aspect is the microneedle patch according to any one of the first to sixth aspects further including a bottom-section layer joining the intermediate layer and the base member, in which the intermediate layer has breaking strength weaker than breaking strength of the top-section layer, and the top-section layer has breaking strength weaker than breaking strength of the bottom-section layer.

A microneedle patch according to an eighth aspect is the microneedle patch according to any one of the first to sixth aspects further including a bottom-section layer joining the intermediate layer and the base member, in which the intermediate layer has a weight-average molecular weight lighter than the weight-average molecular weight of the bottom-section layer, and the bottom-section layer has a weight-average molecular weight lighter than the weight-average molecular weight of the top-section layer.

A microneedle patch according to a ninth aspect is the microneedle patch according to any one of the first to eighth aspects in which the intermediate layer comprises a first intermediate layer made of, as a primary material, a polymer material having a water absorbability lower than the water absorbability of the top-section layer and is adjusted to dissolve in dermis following the top-section layer. The primary material is a material of a content percentage exceeding 50%.

A microneedle patch according to a tenth aspect is the microneedle patch according to the ninth aspect in which the intermediate layer comprises a second intermediate layer made of, as a primary material, a polymer material having a water absorbability higher than the water absorbability of the top-section layer and is adjusted to dissolve in dermis preceding the top-section layer. The primary material is a material of a content percentage exceeding 50%.

A microneedle patch according to an eleventh aspect includes a base member and a plurality of microneedles supported by the base member. Each microneedle includes a top-section layer comprising a biologically active substance to be pierced into dermis, and an intermediate layer provided between the top-section layer and the base member. The intermediate layer is made of, as a primary material, a polymer material having a water absorbability higher than the water absorbability of the top-section layer, and is adjusted to break in dermis in 20 seconds or less.

A microneedle patch according to a twelfth aspect is the microneedle patch according to any one of the first to eleventh aspects in which the biologically active substance is drug.

A microneedle patch according to a thirteenth aspect is the microneedle patch according to any one of the first to twelfth aspects further including a bottom-section layer joining the intermediate layer and the base member, in which the intermediate layer is inside the bottom-section layer.

A microneedle patch according to a fourteenth aspect is the microneedle patch according to any one of the first to thirteenth aspects further including a bottom-section layer joining the intermediate layer and the base member, in which the bottom-section layer has a thickness larger than the thickness of epidermis, and the intermediate layer reaches at the dermis when a surface of the base member contacts the epidermis.

With the microneedle patch according to the first to fourteenth aspects, when the microneedles are administered to skin or mucous membrane, the biologically active substance can be reliably left in the mucous membrane, epidermis, or dermis in a short time. As a result, when the microneedles are administered to skin or mucous membrane, a treatment is reliably completed in a short time.

### Industrial Applicability

A microneedle array manufacturing apparatus according to the present invention provides a technology of fine and mass manufacturing of a microneedle array having highly accurate appearance and accuracy by an inkjet method, provides details of an extremely realistic, reproductive, excellent mass production technology providing a new technology that could not achieve by a conventional method of a technology of controlling the breaking property and internal structure of the microneedle, and relates to development of an apparatus configured to manufacture a microneedle having an excellent breaking property at administration to achieve a desired purpose, thereby allowing fast treatment using a microneedle patch, and having a function to prevent backflow of, through a penetrating hole, drug administered in dermis, thereby providing high industrial applicability in the medical field.

### Explanation of Letters or Numerals

1, 1A apparatus for manufacturing a microneedle array
10, 10A droplet discharging apparatus
11a, 11b, 11c, 11d nozzle
12a first discharge head actuator
12b second discharge head actuator
13a, 13b cartridge
20 positioning apparatus
21 XYZ stage
22 CCD camera
23 alignment monitor
80, 80A, 80B, 80C mold
81 recess
83 alignment mark
85 porous base member
91, 93 top-section-layer raw material liquid
92 bottom-section-layer raw material liquid
100, 100A, 100C, 100D, 100E product including microneedle array
100F, 100G microneedle patch
101 base member
102 surface
103, 103a to 103h microneedle
104, 104a to 104e top-section layer
105, 105a to 105c, 105e bottom-section layer
106, 106c to 106e intermediate layer
1061 first intermediate layer
1062 second intermediate layer
110, 110A to 110E microneedle array

## Claims

1. A microneedle patch produced by a droplet discharging apparatus configured to be capable of separately discharging, as raw material liquid, for example, first liquid, second liquid, third liquid, and fourth liquid containing components different from each other, wherein
an intermediate layer is formed after discharge, drying, and hardening of the top-section layer comprising a biologically active substance, wherein the intermediate layer has a breaking property higher than a breaking property of a top-section layer, has a function to prevent backflow of, through a penetrating hole, drug released in dermis, and has a thickness of 5 µm to 100 µm, and
the intermediate layer is configured to break in the dermis in 5 seconds to 20 seconds approximately.

2. A microneedle patch produced by discharging raw material liquid from a droplet discharging apparatus, wherein the strength of a microneedle is highest in a bottom-section layer, followed in order by a top section comprising drug, and an intermediate layer set as a layer having a lowest strength.

3. A microneedle patch produced by discharging raw material liquid from a droplet discharging apparatus, wherein a weight-average molecular weight of a microneedle is largest in a top section comprising a biologically active substance, followed in order by a bottom-section layer, and an intermediate layer set as a layer having a lowest weight-average molecular weight.

4. The microneedle patch according to any one of claims 1 to 3, which is produced by discharging different kinds of raw material liquid from a droplet discharging apparatus, wherein the intermediate layer consists of two or more layers.

5. A microneedle patch, produced by discharging different kinds of raw material liquid from a droplet discharging apparatus, and including two or more intermediate layers, wherein
a first intermediate layer is formed of a polymer material having a high hardness and a low absorbability and selected as a raw material selected to have high concentration,
a dissolution time of the first intermediate layer is controlled to be 10 minutes to 24 hours, and
drug contained in a top section and released in dermis is prevented from flowing back in a direction toward epidermis through a penetrating hole.

6. The microneedle patch according to claim 5, produced by discharging different kinds of raw material liquid from a droplet discharging apparatus, and including two or more intermediate layers, wherein
a second intermediate layer is formed of a polymer material having a low hardness and a high absorbability and selected as a raw material selected to have low concentration, and
a function to easily break and separate a top section from a bottom-section layer and reliably leave the top section in dermis in a short time is provided.

7. A microneedle patch produced by discharging raw material liquid from a droplet discharging apparatus, wherein an intermediate layer is inside a bottom-section layer.

8. A microneedle patch produced by discharging raw material liquid from a droplet discharging apparatus, wherein
the microneedle patch is formed integrally with a bonding surface or a flat surface adhering to a substrate when part or all of a bottom-section layer is produced, so that
collapse of microneedles due to piercing is prevented, and a top section comprising a biologically active substance easily and reliably breaks from the bottom-section layer, and
a plurality of intermediate layers each having a function to prevent backflow of the biologically active substance from inside of dermis are provided.

9. A microneedle patch produced by discharging raw material liquid from a droplet discharging apparatus, wherein
an intermediate layer is provided between a top section and a bottom-section layer, and
the intermediate layer is configured to break in dermis in 5 seconds to 20 seconds approximately in order to leave the top section in the dermis.

10. A microneedle array manufacturing apparatus for shaping a microneedle array consisting of a plurality of microneedles by filling a plurality of recesses formed in a mold with raw material liquid for forming the microneedles, the apparatus comprising:
at least one droplet discharging apparatus capable of discharging, to each recess, a droplet of the raw material liquid in a predetermined amount equal to or smaller than the volume of the recess; and
a positioning apparatus capable of adjusting relative position between the droplet discharging apparatus and the mold so as to land the droplet into the recess from the droplet discharging apparatus, wherein
the at least one droplet discharging apparatus are a plurality of droplet discharging apparatuses each capable of discharging raw material obtained by changing, as appropriate, a mixture ratio and concentration of a bioabsorbable formulation that forms a microneedle.

11. A microneedle patch manufacturing method comprising the processes of:
forming a top-section layer in a recess of a mold;
discharging, onto the top-section layer in the recess, a plurality of droplets of intermediate-layer raw material liquid from a droplet discharging apparatus;
hardening the intermediate-layer raw material liquid to form an intermediate layer having a breaking strength weaker than a breaking strength of the top-section layer;
discharging, onto the intermediate layer in the recess, a plurality of droplets of bottom-section-layer raw material liquid from the droplet discharging apparatus; and
hardening the bottom-section-layer raw material liquid to form a bottom-section layer having a breaking strength stronger than the breaking strength of the intermediate layer.
